# EUROPEAN PATENT SPECIFICATION

(11) **EP 3 220 913 B1**
(45) Date of publication and mention of the grant of the patent: **08.09.2021**
(21) Application number: 15861304.2
(22) Date of filing: 19.11.2015
(51) Int. Cl.: A61K 31/519

(54) **NANOPARTICLE-BASED VACCINE TARGETING CANCER/TESTIS ANTIGENS (CTA) AND ITS USE IN SOLID AND HEMATOLOGICAL MALIGNANCIES**
NANOPARTIKELBASIERTER IMPFSTOFF GEGEN KREBS/HODEN-ANTIGENE (CTA) UND DESSEN VERWENDUNG BEI SOLIDEN UND HÄMATOLOGISCHEN MALIGNOMEN
VACCIN À BASE DE NANOPARTICULES CIBLANT DES ANTIGÈNES DU CANCER DU TESTICULE (CTA) ET SON UTILISATION DANS LE CADRE DE TUMEURS MALIGNES SOLIDES ET HÉMATOLOGIQUES

(30) Priority: 19.11.2014 US 201462081825 P
(43) Date of publication of application: 27.09.2017
(60) Divisional application: 21180389.5
(73) Proprietor: Kiromic BioPharma, Inc., Houston, TX 77054 (US); College of Pharmacy & Health Sciences, Mercer University, Atlanta, GA 30341 (US)
(72) Inventor: CHIRIVA-INTERNATI, Maurizio, Lubbock, TX 79407 (US); D'SOUZA, Martin J., Atlanta, GA 30341 (US)
(74) Representative: Lucas, Phillip Brian
(86) International application number: PCT/US2015/061703
(87) International publication number: WO 2016/081783

(56) References cited:
- WO-A1-2013/126402
- WO-A2-2012/009611
- US-A1- 2011 123 620
- US-A1- 2011 207 760
- US-A1- 2012 195 962
- US-A1- 2014 328 884
- US-B1- 6 951 657
- CHABLANI LIPIKA ET AL: "Formulation and evaluation of a particulate oral breast cancer vaccine", JOURNAL OF PHARMACEUTICAL SCIENCES, vol. 101, no. 10, October 2012 (2012-10), pages 3661-3671, XP002781221,
- TAWDE S A ET AL: "Formulation and evaluation of oral microparticulate ovarian cancer vaccines", VACCINE 20120817 ELSEVIER LTD GBR, vol. 30, no. 38, 17 August 2012 (2012-08-17), pages 5675-5681, XP002781222, ISSN: 0264-410X
- MIRANDOLA LEONARDO ET AL: "Cancer testis antigens: novel biomarkers and targetable proteins for ovarian cancer.", INTERNATIONAL REVIEWS OF IMMUNOLOGY, vol. 30, no. 2-3, April 2011 (2011-04), pages 127-137, XP002781223, ISSN: 1563-5244
- TAWDE, S ET AL.: 'Formulation And Evaluation Of Oral Microparticulate Ovarian Cancer Vaccines.' VACCINE. vol. 30, no. 38, 17 August 2012, pages 5675 - 5681, XP055444559
- MATTILA JUHA-PEKKA ET AL: "Development of a M cell-targeted microparticulate platform, BSK02(TM), for oral immunization against the ovarian cancer antigen, sperm protein 17.", JOURNAL OF BIOMEDICAL MATERIALS RESEARCH. PART B, APPLIED BIOMATERIALS 04 MAR 2018, 4 March 2018 (2018-03-04), ISSN: 1552-4981
- Maurizio Chiriva-Internati ET AL: "Cancer Testis Antigen Vaccination Affords Long-Term Protection in a Murine Model of Ovarian Cancer", PLoS ONE, vol. 5, no. 5, 12 May 2010 (2010-05-12), page e10471, XP055767077, DOI: 10.1371/journal.pone.0010471

## Description

The present disclosure relates to the field of anti-cancer vaccines, More particularly, the present invention relates to the field of oral anticancer vaccines wherein one or more of the vaccine ingredients are embedded in a matrix in the form of nanoparticles which protects proteinaceous ingredients from gastric attack.

Immunotherapy strategies such as cancer vaccines are considered less toxic and more specific than current treatments available to cancer patients. Due to their specificity, as well as to potent and lasting effects and applicability to virtually any tumor type, anti-cancer vaccines are a focus of interest of clinical oncologists. A critical step in the development of cancer vaccines is the implementation of vaccination strategies that allow for consistent induction of immune responses to tumor antigens (Chiriva-Internati , M et al. PLoS One. 5(5):el0471 (2010); Stagg et al. Methods Mol Med. 64:9-22 (2001). However, despite promising therapeutic potential, there is still a need to overcome major challenges in cancer immunotherapy, including poor immunogenicity of cancer vaccines, off-target side effects of immunotherapeutics, as well as suboptimal outcomes of adoptive T cell transfer-based therapies.

Tawde et al., Vaccine. 2012 Aug 17; 30(38): 5675-5681 reported a study to investigate whether oral vaccination with microparticles containing ovarian cancer antigens could prevent/ retard ovarian cancer growth. A murine ovarian cancer cell line, ID8 was used as a source of antigens as it correlated closely to human ovarian cancer cell lines in signalling pathways and resulted in development of tumor in mice models similar to human ovarian cancer. Despite advancement in vaccine technology, a whole cell lysate vaccine was still considered a very promising approach as it could overcome the perceived demerits associated with a single antigen/epitope vaccine. Murine ovarian cancer ID8 cells were cultured in Dulbecco's Modified Eagles Medium (DMEM) supplemented with 5 µg/ml insulin, 5 µg/ml transferrin and 5 ng/ml sodium selenite (Sigma, St. Louis, MO). The confluent cells were washed with cold phosphate buffered saline (PBS). The flasks were then treated with hypotonic buffer (10mM Tris and 10mM NaCl) and subjected to five 15 min freeze-thaw cycles at temperatures of -80° C and 37° C respectively to obtain the cell lysate. In murine models it was concluded that the efficacy of the vaccine microparticles containing whole cell lysate of ID8 ovarian cancer cells had been demonstrated in retarding tumor growth. Thus, the oral microparticulate vaccine described was considered to provide a promising approach in terms of cost-effectiveness, ease of production and patient compatibility.

Disclosed is an oral anticancer vaccine composition comprising spray-dried microparticles comprising β-cyclodextrin, hydroxypropylmethyl cellulose acetate succinate (HPMCAS), ethyl cellulose, recombinant SP17 protein, *aleuria aurantia* lectin (AAL) and CpG for stimulating an innate immune response,
the β-cyclodextrin, hydroxypropylmethyl cellulose acetate succinate (HPMCAS) and ethyl cellulose comprised in a gastric acid resistant, enteric erodable matrix,
the AAL being present in a microfold cell (M- cell) targeting effective amount, and
the recombinant SP17 protein being encapsulated in the particles and provided in an amount within the range from 1 to 10% w/w based on the weight of the particles.

In further embodiments, the AAL is provided in an amount within the range from about 0.1 to about 0.5% (w/w) based on the weight of the particles.

In some embodiments the CpG is present in an amount within the range from about 0.1 to about 0.5 % (w/w) based on the weight of the particles.

In some embodiments, the vaccine composition further comprises at least one immune response stimulating cytokine. In more specific embodiments, the at least one cytokine is selected from the group consisting of interleukin 2 (IL-2), interleukin 12 (IL-12) or both.

In some embodiments, the vaccine composition further comprises a lysate from said tumor. In more specific embodiments, the lysate is present in an amount providing a lysate protein content (as measured by BCA assay) to the particles within the range from about 1 to about 10 % (w/w) based on the weight of the particles. In further embodiments, the lysate has been prepared by a process comprising use of hypotonic lysis buffer.

In some embodiments, the matrix comprises 60% beta cyclodextrin, 20% HPMCAS and 15% ethyl cellulose.

In some embodiments, the particles of the vaccine composition or of a particle assembly have an average diameter based on particle volume within the range from about 1 to about 5 micrometers. In some embodiments, the particles have a normal size distribution with at least 90 percent of the particles having an average diameter between about 0.5 and 5 micrometers. In some embodiments, the the particles have a zeta potential within the range from about +50 to about -50 mV.

In some embodiments of the particle assembly, the SP17 protein is provided in an amount effective, upon oral administration of the nanoparticles in a vaccine composition to a subject having a malignant tumor expressing said SP 17 protein, to stimulate an immune response against the tumor.

In some embodiments of the particle assembly, the AAL is provided in an amount effective to recruit micro fold cells of said subject.

Other embodiments of the particle assembly have one or more of the features recited for the particles in the vaccine compositions above.

In yet another aspect, the present disclosure relates to a method for treating cancer comprising administering to a subject afflicted with a malignant tumor a vaccine composition as defined above and an aqueous vehicle in which said particles are suspended. The method may comprise repeating the administration at least two and up to 10 times at an interval of 1 to 3 days after the preceding administration and may result in one or more of reducing tumor burden in said subject and prolonging survival of said subj ect.

In embodiments of the method, the subject is afflicted with a malignant tumor selected from the group of solid tumors and hematologic malignancies. In further embodiments, the tumor is a solid tumor selected from the group consisting of ovarian cancer, hepatocellular carcinoma, breast cancer, and prostate cancer or any other solid tumor disclosed in the present specification.

In alternative embodiments, the tumor is a hematologic malignancy selected from the group consisting of multiple myeloma, acute myelogenous leukemia, acute lymphoblastic leukemia, chronic lymphocytic leukemia, chronic myelogenous leukemia, hairy cell leukemia or any other hematologic tumor disclosed in the present specification.

In further embodiments, the method comprises administering to the subject a vaccine composition in an amount within the range between about 5mg to 320 mg said composition containing from about 1 to about 10 % (w/w) of SP17 protein, from about 0.1 to about 0.5 % (w/w) of AAL based on the weight of the particles.

In more specific embodiments, the tumor is ovarian cancer.

In some embodiments of the method, the composition further comprises an adjuvant. In some embodiments of the method, the composition further comprises at least one immunostimulatory cytokine is two cytokines: IL-2 and IL-12.

### BRIEF DESCRIPTION OF THE DRAWINGS

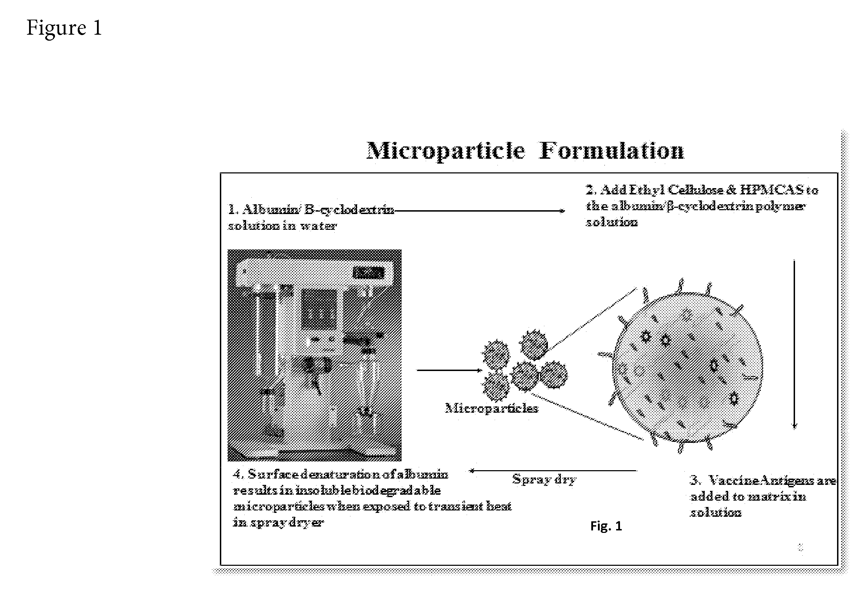

Figure 1 is a schematic representation of steps in microparticle preparation.

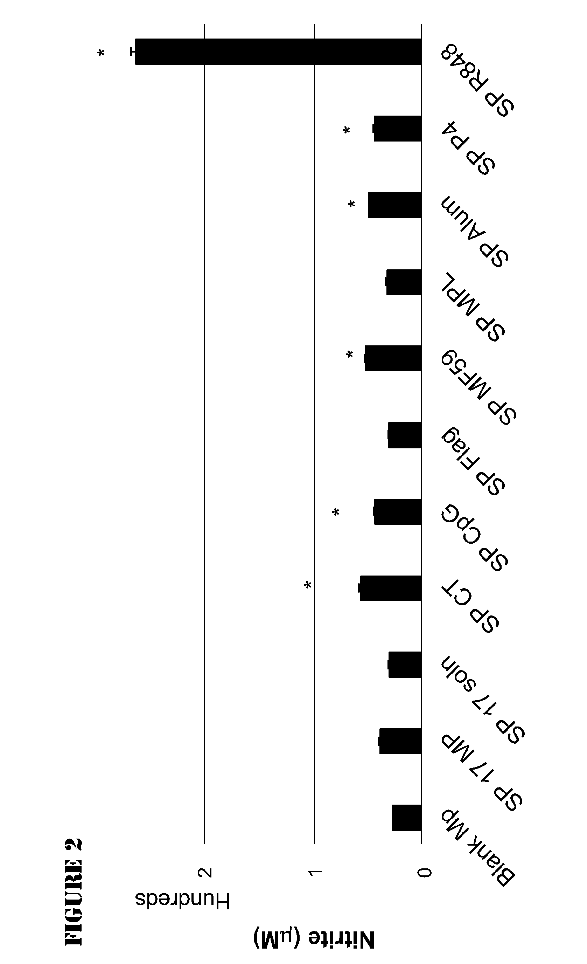

Figure 2 is a bar graph of nitrite (stable physiological reservoir of nitric oxide) release from the dendritic cells (DC) following incubation with respective formulations for 24 hours. The star above the bar indicates statistical significance (p<0.05). All adjuvant treated groups included 7µg of SP17 in microparticles (MP) and 2µg of antigen in MP

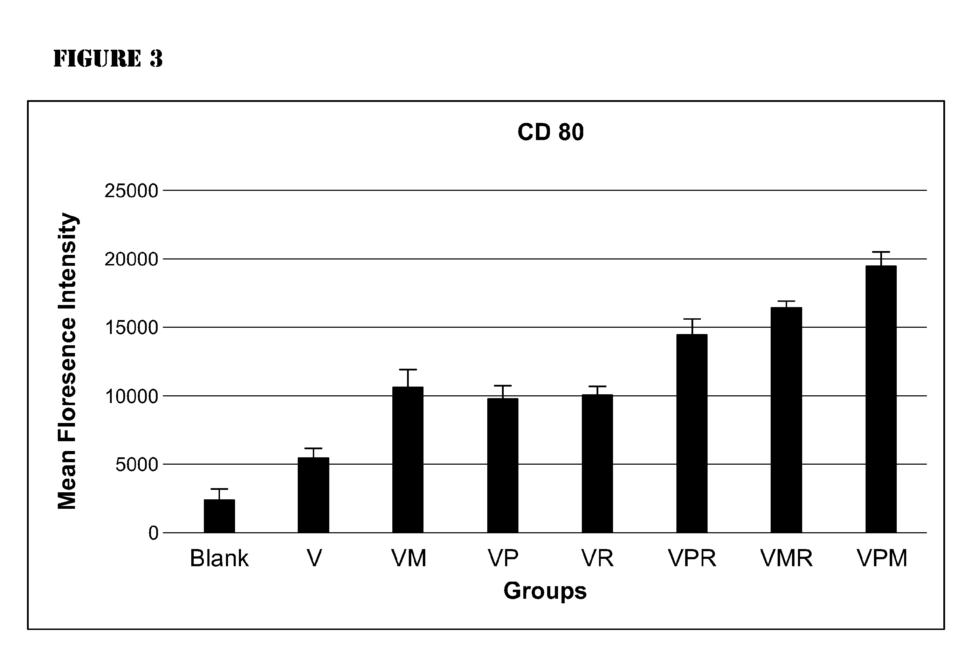

Figure 3 is a bar graph of mean fluorescence intensity indicating CD80 expression on DCs following incubation of microparticle formulations for 16 hours in various adjuvants. Vaccine mp (Vaccine microparticle alone), VM (Vaccine + MF59), VP (Vaccine + P4), VR (Vaccine + R848), VMR (Vaccine+ MF59 + R848), VAM (Vaccine + Alum + MF59), VMP (Vaccine + MF59 + P4), VPR (Vaccine + P4 + R848), and VAP (Vaccine + Alum + P4). Data were obtained using flow-cytometry analysis and CD80 specific antibody. All adjuvant treated groups contained 4µg of antigen and 1µg each of adjuvant in microparticles

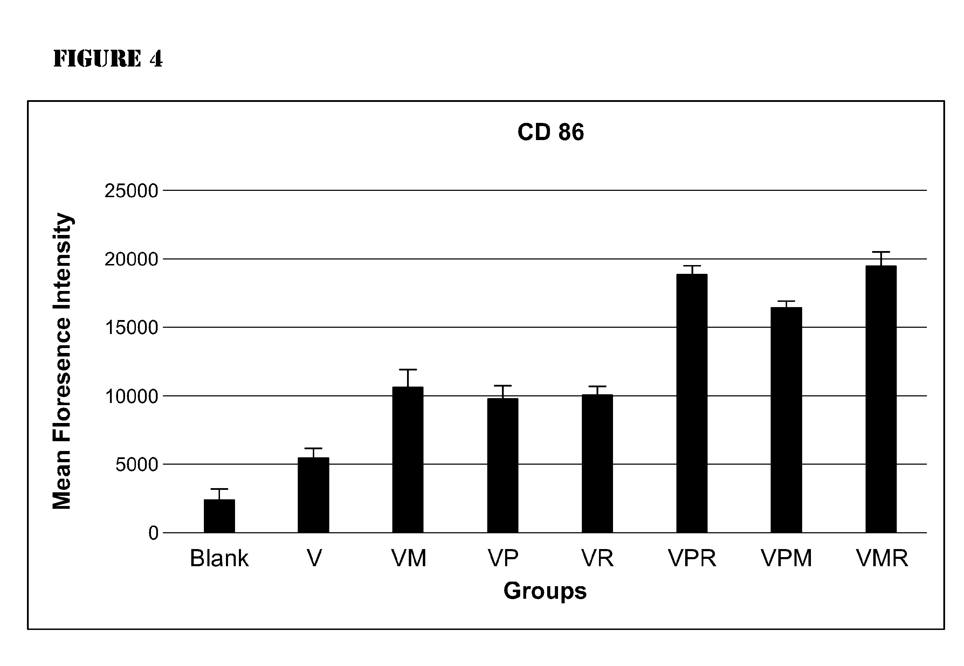

Figure 4 is a bar graph of mean fluorescence intensity indicating CD86 expression on DCs following incubation with microparticle formulations for 16 hours. Data were obtained using flow-cytometry analysis and CD86 specific antibody. All adjuvant treated groups contained 4µg of antigen and 2µg each of adjuvant in microparticles.

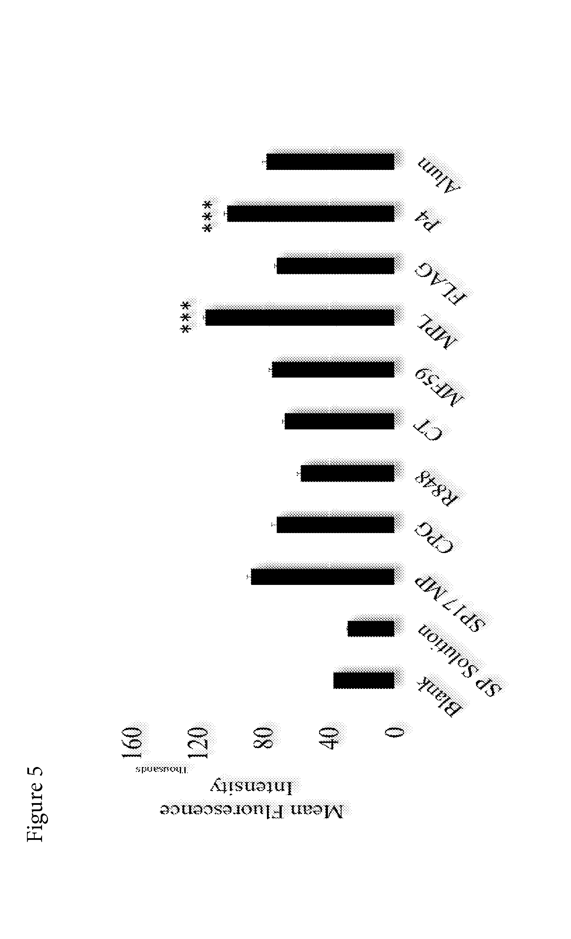

Figure 5 is a bar graph of mean fluorescence intensity indicating CD54 (ICAM-1) expression on dendritic cells (DC) following incubation with microparticle formulations for 24 hours. Data were obtained using flow-cytometry analysis and CD54 specific antibody. All adjuvant treated groups contained 7 µg of SP17 antigen and 2 µg of adjuvant in 1 mg of nanoparticle.

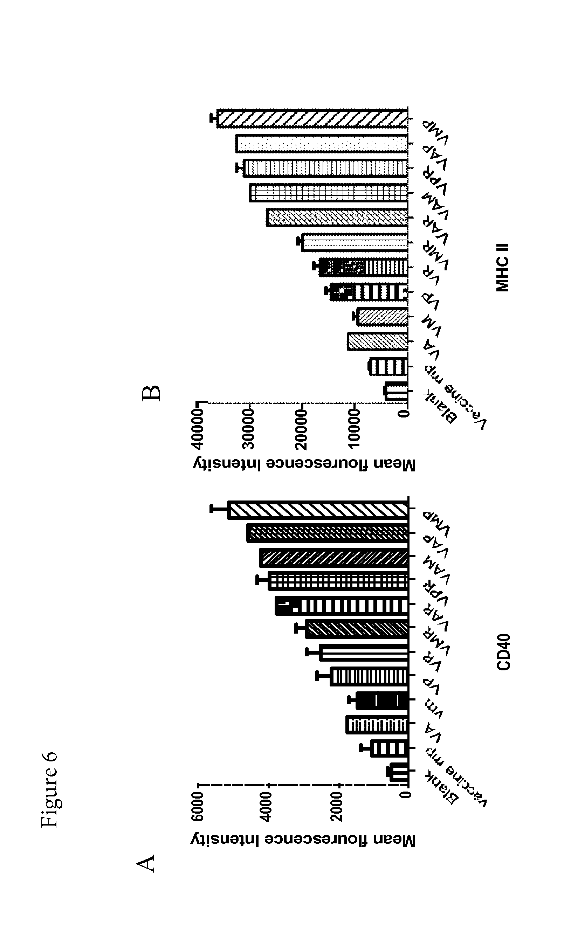

Figures 6A and 6B are two bar graphs of mean fluorescence intensity indicating CD40 (left, 6 A) and MHC II (right, 6B) expression on DCs following the incubation with microparticle formulations for 16 hours. Data were obtained using flow-cytometry analysis and CD40- and MHCII- specific antibodies. All adjuvant treated groups contained 4 µg of antigen and 1 µg of adjuvant in 1 mg of nanoparticle.

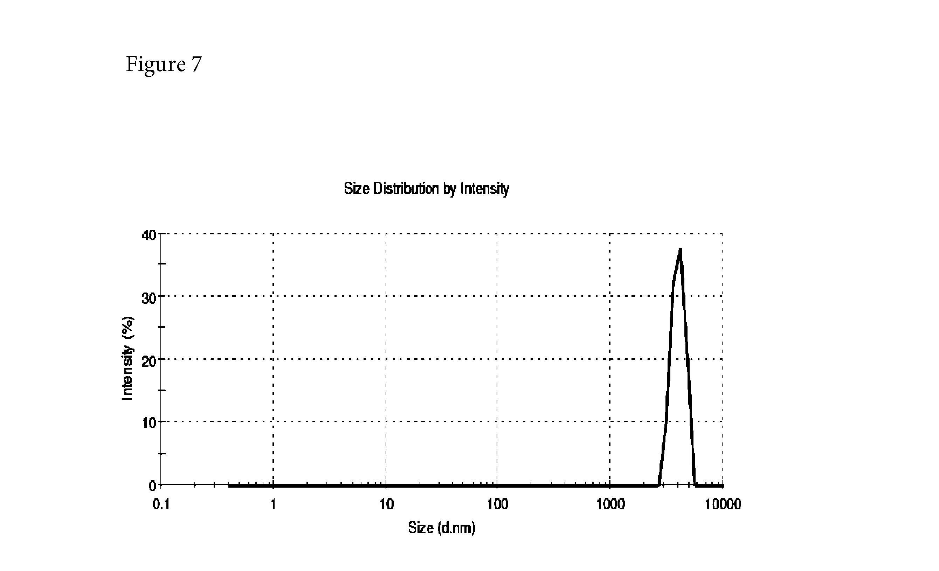

Figure 7 is an intensity weighted particle size distribution graph of SP-17 microparticles obtained using Malvern Zetasizer. SP-17 microparticle size was 3. 59 microns. The zeta potential was determined to be +9.36mV.

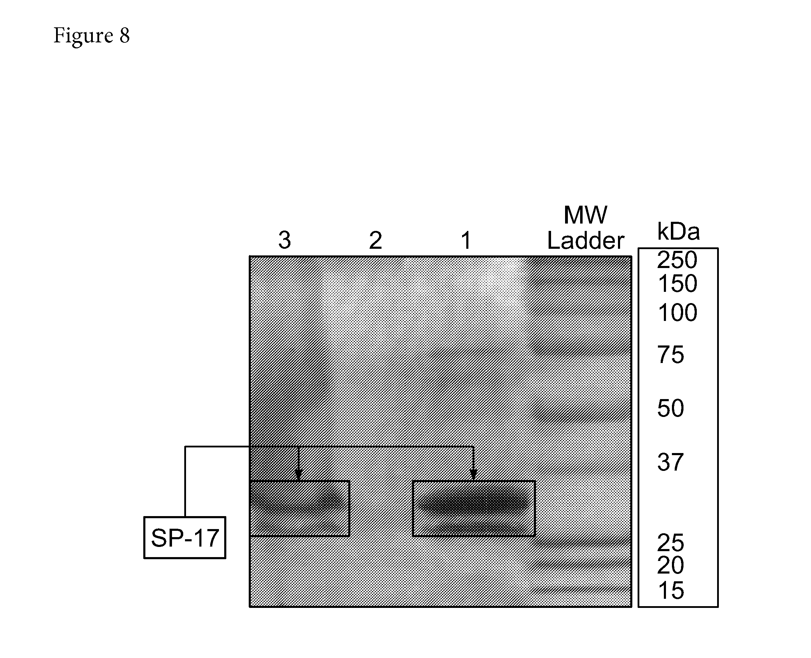

Figure 8 is an image (immunoblot) of sodium dodecyl sulfate polyacrylamide gel electrophoresis (SDS-PAGE) gel indicating stability of SP-17 following spray drying. Lanes from right to left: molecular weight ladder, native SP-17 solution (lane 1), blank microparticles (lane 2), SP-17 in microparticles (lane 3). Microparticle formulations with or without SP-17 were suspended in 100 µl PBS (pH 7) and 30 µl was loaded onto the gel. Blank microparticles were used as a negative control.

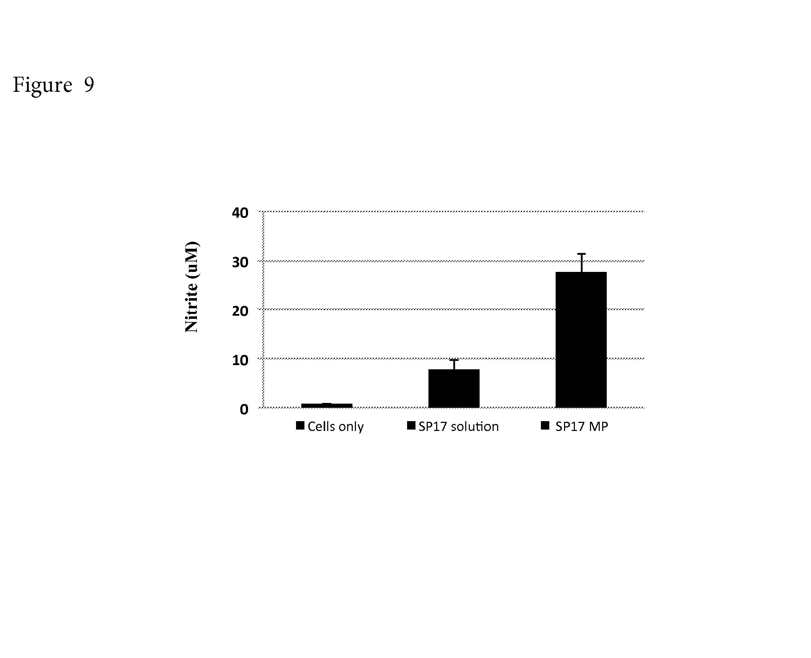

Figure 9 is a bar graph of nitrite (stable physiological reservoir of nitric oxide, in µM) release, which indicates nonspecifically the activation of immune response, in DCs following the incubation with SP17-loaded microparticles or SP-17 in solution (or cells without SP17 treatment). Incubation of DCs with SP17 microparticles led to significant nitrite release compared to DCs incubated with SP17 in solution.

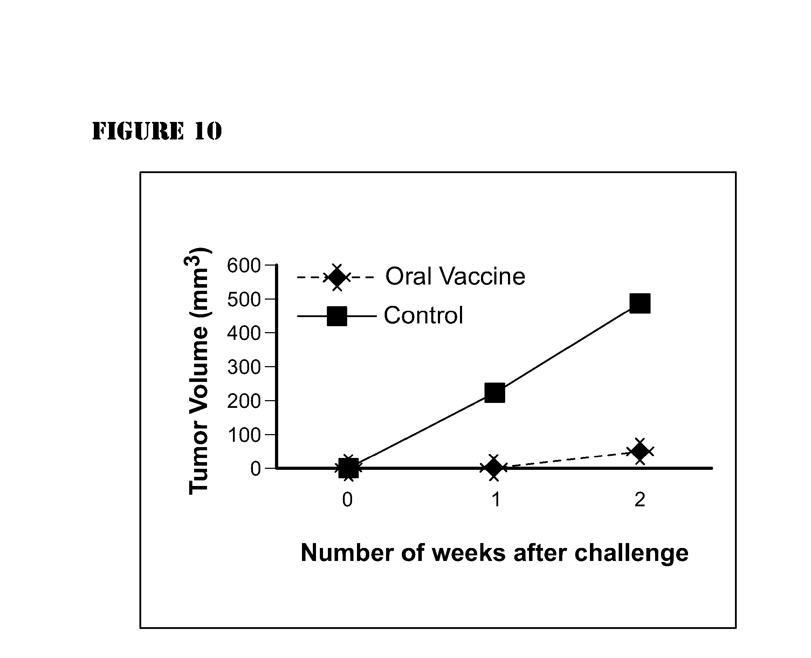

Figure 10 shows that oral vaccination leads to tumor reduction in a breast tumor animal model. The efficacy and potency of oral nanoparticle vaccine was determined using the 67NR breast cancer model. Tumor size was measured after oral vaccination with 67NR murine breast cancer cell lysate followed by challenge with live 67NR cells. Tumor volumes were significantly lower (p<0.01) when compared to the control mice that received blank particles.

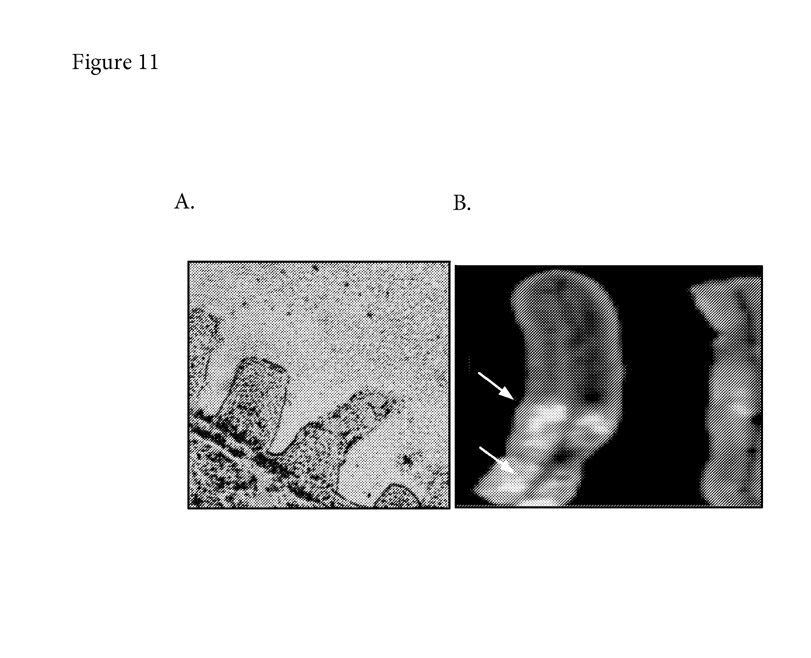

Figures 11A and B show successful uptake of the labeled vaccine particles into the Peyer's patches of mice. A photomicrograph of distribution of blank nanoparticles labeled with a fluorescent dye (left, grey dots), and a photomicrograph of a small intestinal segment (Ileum) in the Peyer's microvillus in the intestine, showing uptake of particles labeled with IR-CW 800 infrared dye into the Peyer's patches (left image).

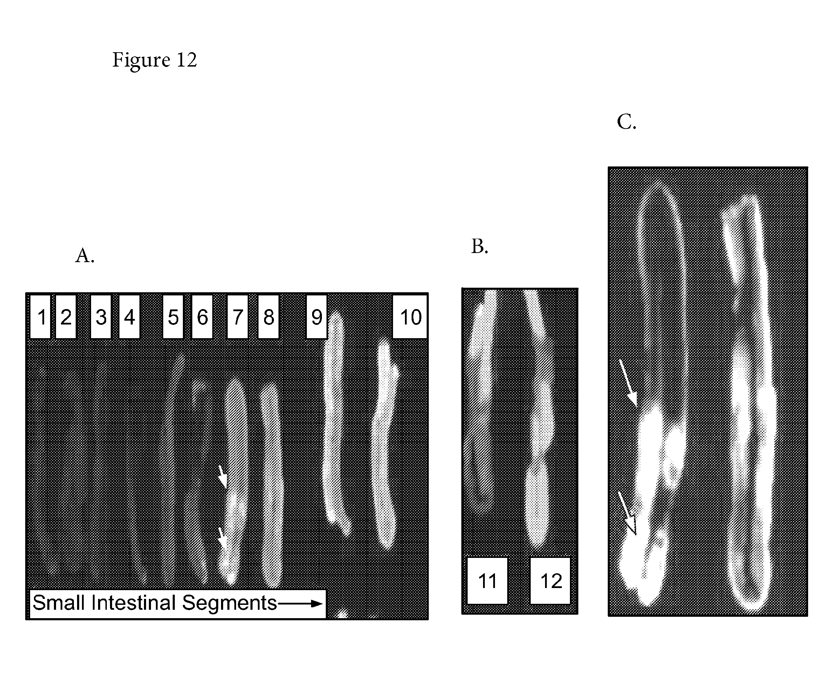

Figure 12A and B show localization of oral nanoparticle vaccine to the Peyer's patches. Rats were administered oral nanoparticle vaccine as described for Figure 1 IB After 6 hours, bio-images of entire small intestines were taken. Initial segments of intestine (jejunum, duodenum, items 1 through 6) did not show any presence of dye, while ileum (items 7 through 12.) showed the presence of dye (shown as white segments). Figure 12C is an enlargement of items 7 and 8 from Figure 12A. Details of Peyer's patches are visible.

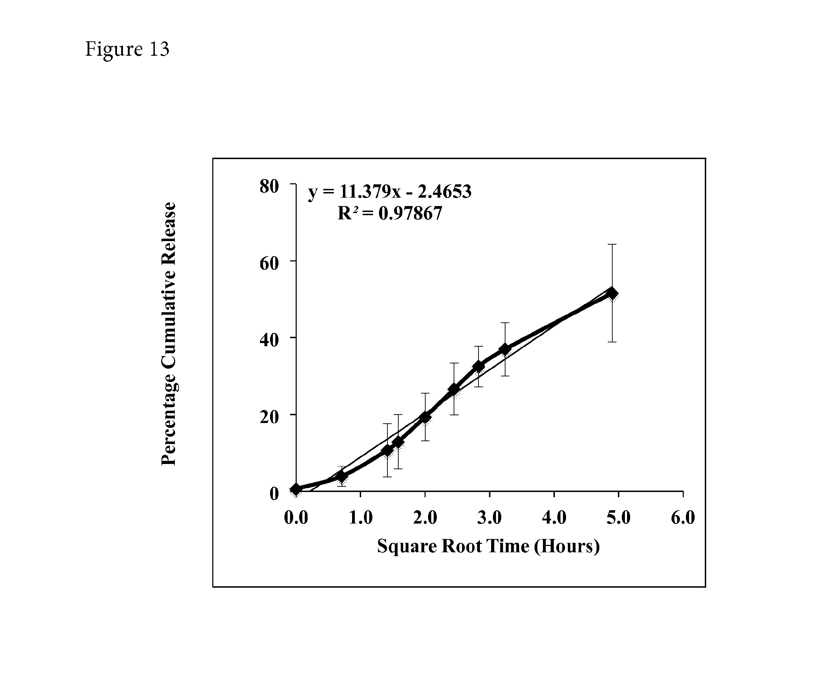

Figure 13 is a plot of percent cumulative release of antigen against the square root of time from administration, depicting a release profile of antigen from β-cyclodextrin matrix particles. The best line fit y = f(x) and slope R² are included. (This figure was published in Chablani et al. J Pharm Sci. 101(10): 3661-71. (2012)).

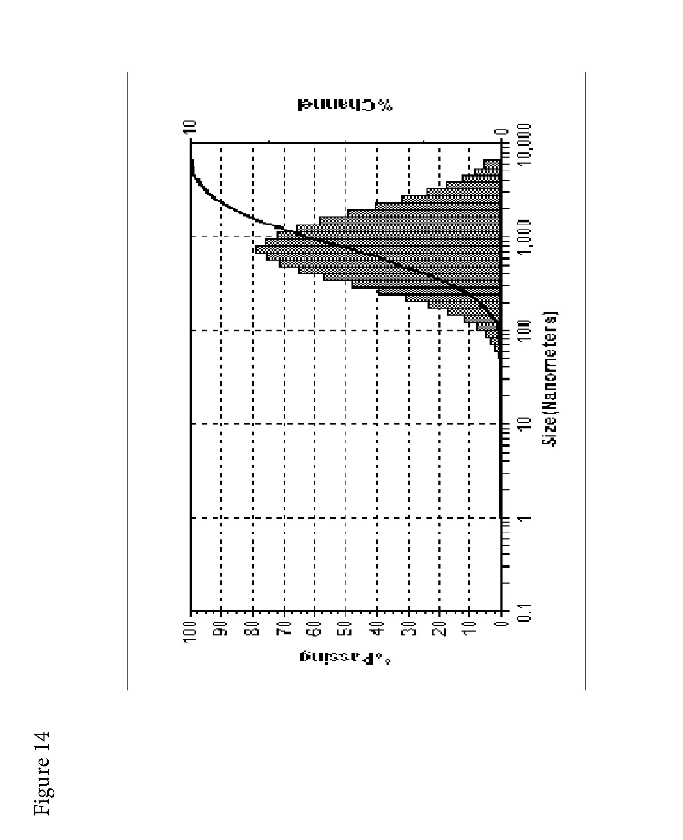

Figure 14 is a size distribution plot of a batch of microparticles according to the present disclosure. The microparticles had an average particle size of 1.1 microns and zeta potential of -31.8 mV. The left ordinate axis represents percentage of particles passing a given mesh (thereby providing a size cutoff value and the results are plotted as an S-curve) and the right ordinate axis represents the percentage of volume-based particle size. The measurements were generated using a Zetatrack Ultra analyzer.

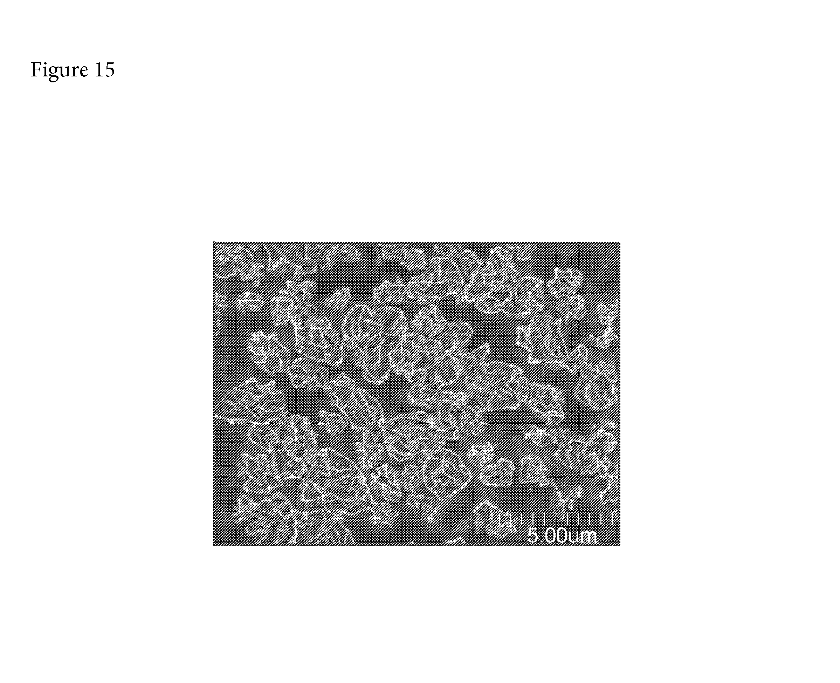

Figure 15 is a microphotograph (taken by scanning electron microscopy) of vaccine microparticles produced by spray drying. A scale of 5 micron increments is included.

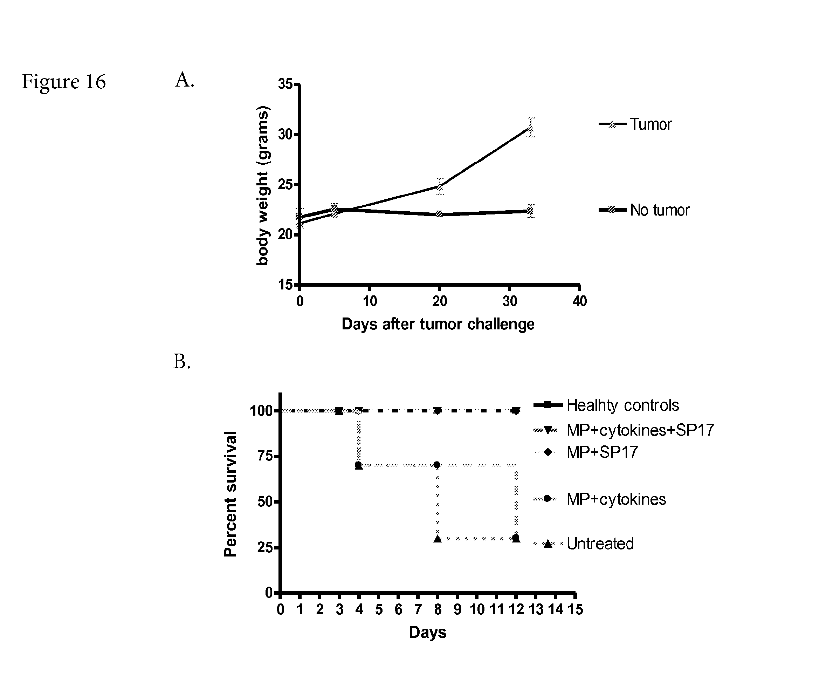

Figure 16A is a plot of mouse body weight over time (with an increase indicative of implanted ID8 tumor growth) in an ongoing experiment; Figure 16B is a plot of percent mouse survival over time for each of healthy controls (black squares), mice implanted with ID8 tumor cells but later vaccinated with microparticles plus SP17 with cytokines (IL-2 and IL-12, black downward pointing triangles) or with microparticles plus SP17 without cytokines (black diamonds) or with microparticles plus cytokines but without SP17(black circles) or left untreated (black upward pointing triangles) (n=4 in each group). After 12 days, survival of the MP + SP17 vaccinated animals even without cytokines was still indistinguishable from healthy controls whereas all but one mouse in each control group died.

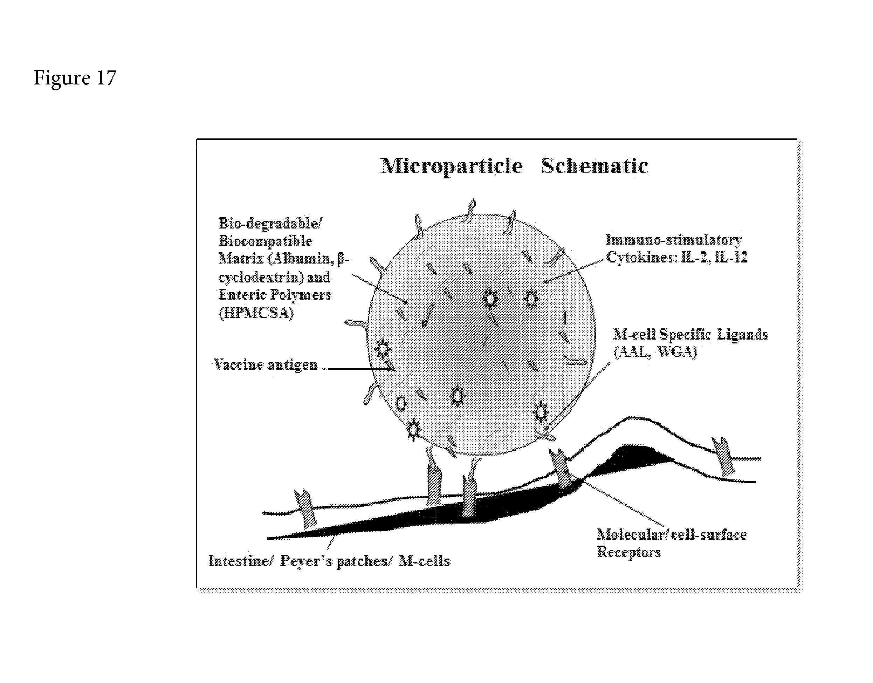

Figure 17 is a general scheme of vaccine compositions embodiments of the present disclosure.

### DETAILED DESCRIPTION

The present particles assembly and vaccine compositions incorporating such particles represent the first use of SP17 protein in an oral vaccine. It had previously been shown that that CTA are expressed by solid and hematological malignancies, regardless of the histological origin of the tumor and of the differentiation stage^{1,18}. For instance, the present inventors and co-workers have previously discovered and validated a novel CTA panel, including SP17,^{1,,3,5-9,19-26} Ropporin,^{4,5,25} AKAP4,^{5,10,25,27,28} PTTG1,^{25,29,30} which have so far led to the successful pre-clinical validation of these antigens in different human tumors, namely OC,^{3,9,11,31,32}MM,^{4,7,8,14,24,28}PC,¹⁰ and LC.³³

Combining SP17 protein with nanoparticle-based oral vaccine delivery represents an approach that has never been attempted before and that addresses many of the problems associated with current vaccine therapies, such as dendritic cell vaccines, including their high cost. Oral vaccine delivery as proposed is an attractive mode of immunization because of its ease of administration, low manufacturing cost and patient acceptability. Generally, orally delivered particulate vaccines are recognized by micro fold (M) cells, which sample the antigen in the Peyer's patches of the intestine and pass them on to professional antigen presenting cells (APCs) such as dendritic cells and macrophages that are present in the Peyer's patches³⁴. These APCs then phagocytose, process and present the particulate antigens on both MHC Class I and MHC Class II molecules to T-cells. Since dendritic cells have receptors for both IL-12 and IL-2^{5,6,7} and have the capacity to present exogenous antigens to both Class I (cross- presentation) and Class II pathways⁴, the inventors anticipate expect that both T-cell subsets will be shown to be activated by the present vaccination methods using the particulate vaccine compositions disclosed here. Accordingly, the immunostimulatory cytokines IL-2 and IL-12 are also included in some embodiments.

Encapsulation of proteins into biodegradable polymer particles has been shown to increase the amount of antigen presented by dendritic cells to the T-cell compartment by facilitating the endosome escape of encapsulated antigens within the APCs. This resulted in high efficiency MHC-Class I presentation by APCs and the accompanying tumoricidal CD8+ CTL response³⁵. To enhance the targeting capability of the nanoparticle vaccine formulation to M-cells in the Peyer's patches of the intestine, the inventors have incorporated the M-cell targeting agent, Aleuria aurantia (AAL), into vaccine particles. It has recently been shown that AAL targets nanoparticles to M-cells very efficiently³⁶. Novel nanoparticle vaccine of the present disclosure also addresses the obstacle represented by immune suppressor regulatory T- cells (T-reg) in human malignancies. This innovative vaccine formulation will reduce the T-reg response as indicated by several lines of evidence using M-cell-targeting vaccines³⁷. Indeed, the present inventors anticipate that the nanoparticulates are efficiently taken up by M-cells, and then transferred to mucosal DCs. These are then anticipated to differentiate towards a CTL-polarizing profile, thus skewing the CTL/Treg balance³⁸. Although the detailed mechanism has still to be elucidated, it is known that M-cell targeting vaccines have the potential to attenuate or impair the T-reg response, making this strategy highly promising as an addition to anti-tumor therapies⁴⁰. This effect is postulated to be increased by the addition of the TLR- stimulating CpG or another adjuvant¹⁵. It is also anticipated that addition of IL-2 and IL- 12 to the oral nanoparticles will be shown to induce a more evident antibody response without inducing any toxicity. In vitro toxicity studies with cultured cells showed no toxicity even at very high concentrations. General features of the vaccine composition of the present disclosure are depicted in Figure 17 which is a schematic representation of vaccine embodiments including some of the present disclosure.

The foregoing nanoparticle formulation has been previously evaluated for oral delivery of other vaccine antigens such as typhoid antigen⁴⁶, tuberculosis antigen⁴⁷, prostate cancer vaccine⁴⁸, and insulin⁴⁹. No toxicity was observed.

In addition, CTA targeted vaccines have been evaluated for toxicity in animal models and found safe. See reference 43 below. The CTA vaccine described in this reference showed no toxicity related to the vaccine in syngeneic mice. No mice exhibited macroscopic toxicities and post-mortem evaluation of organs (liver, spleen, lungs, kidney, heart, bone marrow) did not evidence any alteration related to the vaccine. The vaccine consisted of an injection of full-length recombinant SP17 protein mixed with the CpG adjuvant. The formulation shares antigen and adjuvant with the one used in experiments herein but without the use of nanoparticles. Such injection formulation did not produce any acute or chronic toxicity for the entire duration of the experiments, 300 days, in vaccinated mice, and therefore the chances of oral nanoparticle-related toxicities are dramatically low. This is so especially since the doses administered with the oral microparticulate formulation are much lower (10 times lower for SP17 and 50% lower for CpG) than those given by injection.

Moreover, Dr. D'Souza's previously published studies show that high concentrations of nanoparticulate vaccine are not toxic for macrophages and non-tumoral cells in vitro. In vitro cyctoxicity assay showed that the nanoparticles are not toxic to the cells. RAW 264.7 (murine macrophage) cells were incubated with various concentrations of nanoparticles for 24 firs and it was found that the nanoparticles were not toxic up to 2 mg/ml concentration. Furthermore, in another published study by Dr. D'Souza, where murine breast cancer cells 4T07 were used as a source of antigens for the preparation of the microparticle vaccine, cytotoxicity studies showed no cytotoxicity at all the concentrations tested (Chablani et al. J Pharm Sci. 101(10): 3661-71. (2012)).

In another study by the same group involving primates, administration of nanoparticles (without CTA) did not affect any normal physiological parameters. No allergic reactions or antibodies against the formulation were detected<3>. These results show that our proprietary microparticulate formulation is non-toxic and highly safe even in large animals (primates). Therefore, toxicities of the present vaccine compositions in vivo are unlikely.

### Particles and Preparation

As used herein, the terms "microparticles" and "nanoparticles" are used interchangeably in the sense that what is said about one can also apply to the other. The term "particles" is used to denote microparticles or nanoparticles, as those term are used in the art, or mixtures thereof. Typically, microparticles have an average (mean) diameter range between about 1 and about 5 microns.

Particles and therefore particle assemblies in accordance with the present disclosure can be made by spray drying methods, preferably in one step, by mixing the various matrix and active ingredients, in an aqueous medium spraying in droplets of controlled size and drying the droplets to form microparticles or nanoparticles or both. Detailed methods and ingredients for making microparticles in accordance with the invention is disclosed in PCT Patent Application PCT/US2009/058896 published as WO 2010/037142. See the entire disclosure, (including the claims) which is incorporated by reference in its entirety and especially see pp. 48 to 56. Physical characteristics of the particles (such as size morphology, distribution and zeta potential and any other characteristic assessed in the pharmaceutical API or formulation particle art) can be assessed by any known method, for example by methods described below in the Examples. The ingredients for the particle matrix include a water soluble polymer such as beta cyclodextrin and enteric coating materials such as ethyl cellulose (EC) and hydroxypropylmethyl cellulose acetate succinate (HPMCAS). In some embodiments, the matrix ingredients form approximately 80 to 95% of the particles, with the active ingredients forming approximately 5 to 20%.

In some embodiments, approximate relative amount ranges (w/w) of the matrix ingredients based on the weight of the particles are as follows:
Beta-cyclodextrin: 40 to 80 %;
Combined EC and HPMCAS: 10 to 35 %
For example, in some embodiments, the approximate amount of ethyl cellulose is from 0 to 15 % and that of HPMCAS is 10 to 35 %.

More details on the approximate relative amounts of the active ingredients SP17 protein, lectin, cytokine, adjuvant and tumor lysate proteins follow in each section. Of these, each one of cytokine, adjuvant and lysate and combinations of two or all three of them are preferably included as will be illustrated below.

In a particular formulation employed in the present experiments the matrix contains approximately 60% beta cyclodextrin, 20% HPMCAS, 15% EC. The remainder is CTA. The optional ingredients are added to this formulation in percentages within the ranges disclosed herein.

The nanoparticles having a particular formulation of 60% beta cyclodextrin, 30%) HMPCAS, 15%) EC used in the experiments described in the examples have been evaluated.

### Cancer Testis Antigens (CTA)

The present disclosure features the use for the first time in an oral vaccine of SP17 protein.

### Lectins

The lectin that may be used in the methods, vaccine compositions and particles of the present disclosure is Aluria aurantia lectin (AAL). [0040] The amount of lectin or lectins that is typically used in the present particles, vaccine compositions and methods is within the range of about 0.1 to about 0.5% (w/w) based on the weight of the particles.

### Adjuvants

Adjuvants suitable for use in the methods, compositions and particles of the present disclosure include without limitation any pharmaceutically acceptable adjuvant that will stimulate the initial innate immunity response to antigen (SP17 and tumor lysate protein if the latter is used) and thus potentiate immunogenicity of SP17 given by oral route. CpG is included in the composition as also may be MF59, alum, flagellin, R848, monophosphoryl lipid A, ODN 1826, and combinations of at least two of the foregoing. Even if an adjuvant does not potentiate the immune response with a particular vaccine and a particular CTA, as long as it has been demonstrated to have adjuvant activity in at least one system, that adjuvant is potentially useful in another embodiment of the present disclosure.

In the Examples below, CpG was used as adjuvant when an adjuvant was used as it has been been shown to provide signals for dendritic cells and T-cell activation^{41,42} and has been shown to provide an efficient adjuvant effect in oral vaccines formulations⁴⁵.

The present inventors have screened the following adjuvants as potentiators of the present particles and vaccine compositions:
AddaVax™(MF59) is a squalene-based oil-in- water nano-emulsion based on the formulation of MF59 that has been licensed in Europe for adjuvanted flu vaccines. Squalene is an oil more readily metabolized than the paraffin oil used in Freund's adjuvants. AddaVax™ promotes a significant increase in antibody titers with reportedly more balanced Th1/Th2 responses than those obtained with alum. MF59is available from Novartis AG. AS03 (Adjuvant System 03) is another squalene-based adjuvant commercially available from Glaxo SmithKline. A dose of AS03 adjuvant contains 10.69 mg squalene 11.86 mg DL-a-tocopherol .86 mg polysorbate 80.

Alhydrogel 2% is an aluminium hydroxide (referred to as alum) wet gel suspension. Alum improves attraction and uptake of antigen by APCs. It has been suggested that the antigens absorbed on the aluminum salts are presented in a particulate form, making them more efficiently internalized by APCs. Alum increases Th2 response (leading to antibody production) but does not promote significant Th1 cellular response.

Flagellin (FliC) is a recombinant flagellin protein encoded by the fliC gene from Salmonella typhimurium. Unlike other TLR agonists, flagellin tends to produce mixed Th1 and Th2 responses rather than strongly Th1 responses. It has been demonstrated that flagellin can act as a potent adjuvant in flu vaccines.

R848 is imidazoquinoline compounds and agonists for TLR7 and TLR8. They are effective adjuvants by activating dendritic cells (DCs) and B cells to induce cytokines optimal for Th1 cell immunity, and antibody production.

MPLA (monophosphoryl lipid A), a TLR4 agonist, is a derivative of lipid A from Salmonella minnesota R595 lipopolysaccharide (LPS or endotoxin). MPLA is considerably less toxic than LPS whilst maintaining the immunostimulatory activity. When tested in animals models as a vaccine adjuvant, MPLA induces a strong Th1 response.

ODN 1826 are synthetic oligodeoxynucleotides containing unmethylated CpG motifs (CpG ODNs). CpG ODNs are recognized by TLR9, which is expressed exclusively on human B cells and plasmacytoid dendritic cells (pDCs), thereby inducing Th1 -dominated immune responses. Pre-clinical and clinical trials have demonstrated that CpG ODNs can significantly improve vaccine-specific antibody responses. ODN1826 is commercially available, e.g., from InvivoGen, San Diego CA 92121 (website domain: invivogen.com)

P4 is a 28-30 kDa lipoprotein present in all typeable and nontypeable strains of H. influenzae. P4 is an attractive adjuvant; however, the ability of P4 to induce antiboides protective against infections is still controversial. One P4 isolate has the deduced amino acid sequence GenBank accession no. P26093.

The amount of the at least one adjuvant that is typically used in the particles, vaccine compositions and methods of the present disclosure is within the range from about 0.1 to about 0.5 % (w/w) based on the weight of the particles.

### Cytokines

CpG which has been shown to enhance the efficacy of weak tumor antigens and promote T cell responses^{42,43} is, as explained above, included in the present composition. Other cytokines such as IL-2 and IL-12 which have been shown to provide signals for dendritic cells and T-cell activation are advantageously included in the present CTA-containing oral vaccine compositions, particles and methods. As the present inventors and their co-workers have recently described with oral vaccines using lysates of the murine ovarian cancer ID8 cells as autologous (without additional CTAs) targets.

### Tumor Cell Lysate Antigens

The immune response to cancer is largely determined by the way in which tumor cells die. As necrotic, stress-associated death can be associated with activation of antitumor immunity, whole tumor cell antigen loading strategies for dendritic cell (DC)-based vaccination have commonly used "necrotic" lysates as an immunogenic source of tumor-associated antigens. Thus, due to the presence of many relevant immunogenic epitopes, whole tumor lysates are promising antigen sources for dendritic cell (DC) therapy, as such immunogenic epitopes can help prevent tumor escape.

In one embodiment, the compositions of the present disclosure may include one or more tumor-associated antigens, where tumor-associated antigens can be provided by a tumor cell lysate. Preferably, an autologous tumor cell lysate is provided, i.e. a lysate from a tumor derived from the patient to be treated. However, it is also possible to use tumor-associated antigens from a tumor cell line. In that event, preferably, a tumor cell line of the same tumor type as the tumor to be treated is used.

Any method suitable for lysate preparation can be used in preparation of vaccine microparticles of the present disclosure. Two common methods of whole tumor cell lysate preparation are ultraviolet B (UVB) ray-irradiation and repeat cycles of freezing and thawing. More recently, hypochlorous acid (HOCl)-oxidation method has also been used for inducing primary necrosis and enhancing the immunogenicity of tumor cells. Since vaccine efficacy can vary depending on the method of lysate preparation used, vaccine formulations containing lysates prepared by different lysis protocols (freeze-thaw, UVB, or HOCI) can be assessed in order to determine which protocol provides optimal vaccine efficacy.

While variations can be introduced into each cell lysis protocol, as is appreciated by and known to those skilled in the art, the following exemplary protocols illustrate general features of lysis procedures.

Freeze-thaw method: The confluent cells are washed with cold phosphate buffered saline (PBS). The flasks are then treated with hypotonic buffer (lOmM Tris and lOmM NaCl) and subjected to five 15 min freeze-thaw cycles at temperatures of -80 °C and 37° C respectively to obtain the cell lysate. This protocol was used in the experiments described in the Examples below when tumor lysate was used.

UVB irradiation method: For UVB-irradiation, confluent cells are subjected to a UVB-irradiation for 10 min to induce apoptosis. Cells are then incubated overnight at 37C, 5% C02, and harvested on the following day for 5 cycles of freeze-thaw treatment (for example, freezing with dry ice for 20 min and thawing at room temperature) before use.

HOCI oxidation method: HOC1 solution is prepared by diluting the stock sodium hypochlorite solution (NaOCl, reagent grade, available chlorine 10-15%; Sigma- Aldrich Corp.) with DPBS (Cellgro) and added immediately to tumor cells. The tumor cell suspension is then incubated for 1 h at 37°C, 5% CO₂ with gentle agitation after every 30 min to induce oxidation-dependent tumor cell death. After that, tumor cells are harvested, washed twice with DPBS and resuspended at 1 x 10⁷ cells/ml in appropriate DC media for 6 cycles of freeze (either with dry ice for > 20 min or at -80°C for > 1 h) and thaw at room temperature to complete fragmentation before loading onto DCs.

Various methods of the whole lysate preparations and their advantages are discussed by Chiang et al. in Semin Immunol. 22(3): 132-143 (2010) and in Vaccines. 3, 344-372 (2015).

### Tumor Types

In one embodiment, the present disclosure relates to methods for treating a patient afflicted with a malignant tumor. In some embodiments, the tumor is a solid tumor. In other embodiments, the tumor is a hematologic tumor.

In some embodiments, the tumor is selected from the group consisting of adrenal (e.g., adrenocortical carcinoma), anal, bile duct, bladder, bone (e.g., Ewing's sarcoma, osteosarcoma, malignant fibrous histiocytoma), brain/CNS (e.g., astrocytoma, glioma, glioblastoma, childhood tumors, such as atypical teratoid/rhabdoid tumor, germ cell tumor, embryonal tumor, ependymoma), breast (including without limitation ductal carcinoma in situ, carcinoma, cervical, colon/rectum, endometrial, esophageal, eye (e.g., melanoma, retinoblastoma), gallbladder, gastrointestinal, kidney (e.g., renal cell, Wilms' tumor), heart, head and neck, laryngeal and hypopharyngeal, liver, lung, oral (e.g., lip, mouth, salivary gland) mesothelioma, nasopharyngeal, neuroblastoma, ovarian, pancreatic, peritoneal, pituitary, prostate, retinoblastoma, rhabdomyosarcoma, salivary gland, sarcoma (e.g., Kaposi's sarcoma), skin (e.g., squamous cell carcinoma, basal cell carcinoma, melanoma), small intestine, stomach, soft tissue sarcoma (such as fibrosarcoma), rhabdomyosarcoma, testicular, thymus, thyroid, parathyroid, uterine (including without limitation endometrial, fallopian tube), and vaginal tumor and the metastasis thereof. In some embodiments, the tumor is selected from the group consisting of breast, lung, GI tract, skin, and soft tissue tumors. In some further embodiments the tumor is selected from the group consisting of breast, lung, GI tract and prostate tumors.

In some embodiments, the tumor is selected from the group consisting of leukemia, lymphoma, and myeloma. In some further embodiments, the tumor is selected from the group consisting of myeloid leukemia (AML), acute lymphocytic leukemia (ALL), chronic myelogenous leukemia (CML), non-Hodgkin lymphoma (NHL), Hodgkin lymphoma, chronic lymphocytic leukemia (CLL), and multiple myeloma.

### Vaccine Unit Dosage and Administration Including Booster Schedule

In one embodiment, the dose of the oral vaccine for the use in mice is in the range of 50 g -5 mg /dose/mouse, where the upper limit dose of 5 mg/dose/mouse is based the ongoing animal experiments and then may end-up being higher or lower within the same order of magnitude. In another embodiment, the dose of the oral vaccine for the use in mice is in the range of 100 g - 4 mg/dose/mouse. In a more specific embodiment, the dose of the oral vaccine for the use in mice is in the range of 500 g - 2.5 mg/dose/mouse.

In some embodiments, the vaccine is administered as one prime dose followed by several booster doses. The number of booster doses is in the range of 1-10 booster doses. In one embodiment, the vaccine is administered as one prime followed by 2-8 booster doses. In another embodiment, the vaccine is administered as one prime followed by 3-7 booster doses. In a more specific embodiment, the vaccine is administered as one prime followed by 6 booster doses.

In one embodiment, booster doses are administered at intervals within the range fom about every 5 days to about 3 weeks. In another embodiment, a booster dose is administered every 2 weeks or even weekly.

In one embodiment, the dosage of adjuvant administered to mice is in the range of 2-30 g/dose/mouse. In another embodiment, the dosage of adjuvant is in the range of 5-25 g/dose/mouse. In a further embodiment, the dosage of adjuvant is in the range of 7.5-20 µg/dose/mouse.

In one embodiment, the dose of the oral vaccine for the use in human subjects is in the range of 5mg to 320 mg. In one embodiment, the oral vaccine is administered at an initial dose of 5 mg to a human subject, after which the dosage is escalated to 320 mg per dose (320 mg per dose will be the final dosage in the phase I safety trial). It is anticipated that depending on the results obtained during the phase I study, the dosage requirements may change.

In another embodiment, the dose of the oral vaccine for the use in human subjects is in the range of 10 mg to 250 mg. In further embodiment, the dose of the oral vaccine for the use in human subjects is in the range of 20 mg-200 mg. [

In one embodiment, the oral vaccine of the present disclosure is administered to a human subject as one prime dose followed by several booster doses. In one embodiment, the number of booster doses varies in the range between 1-10 booster doses during the course of treatment. In one embodiment, the vaccine is administered as one prime followed by 2-8 booster doses. In another embodiment, the vaccine is administered as one prime followed by 3-7 booster doses. In a more specific embodiment, the vaccine is administered as one prime and 6 booster doses.

In one embodiment, one or more booster doses are administered at intervals within the range of 1-3 weeks. In another embodiment, a booster dose is administered every 10 to 18 days. In a preferred embodiment, a booster dose is administered every 2 weeks.

In one embodiment, the dosage of adjuvant administered to a human subject is in the range of 50-500 µg/dose/human. In another embodiment, the dosage of adjuvant administered to a human subject is in the range of 100-400 µg/dose/human. In a further embodiment, the dosage of adjuvant administered to a human subject is in the range of 100-400 µg/ dose/human.

### EXAMPLES

### Example 1

### Preparation of Vaccine Nanoparticles

The inventors have developed a biodegradable and biocompatible polymer matrix system for oral vaccines composed of β-cyclodextrin (60%), hydroxypropylmethylcellulose acetate succinate (HPMCAS, 20%), and ethyl cellulose (EC, 15%). The HPMCAS was incorporated because of its enteric properties to protect the proteins from the harsh gastric conditions. By the way of general description of the particles, the β-cyclodextrin and ethyl cellulose function as the sustained release polymer matrix. These compounds are regarded as safe (GRAS) by the FDA for human use. The oral vaccine formulation contained one or more cancer testis antigens (CTA) or miscellaneous tumor-derived antigens prepared from whole tumor cell lysates. Furthermore, the vaccine formulation also contained microfold (M)-cell targeting ligand Aleuria Aurantia lectin (AAL, 0.25% w/w loading), which has been shown to improve the targeting of the particles to the Peyer's patches. Generally, M cells act as sampling ports for any foreign molecules encountered in the small intestine, and have been shown to house numerous dendritic cells and immune cells. Following the sampling of the oral vaccine by M cells, the particle is processed by a dendritic/antigen presenting cell (APC) and presented on major histocompatibility complex (MHC) class I or MHC class II molecules. CpG oligodeoxynucleotides, which have been shown to provide signals for the dendritic cell and T- cell activation were used as an adjuvant in the formulation (Emtage et al. J Interferon Cytokine Res. 18(11): 927-937 (1998)). CpG has also been shown to enhance the efficacy of weak tumor antigens and to promote T cell responses (Zhang et al. J Immunother. 30(5) 469-47% (2007), Chiriva-Internati et al. PLoS One. 5(5):el0471 (2010)). Furthermore, the vaccine formulations described herein also contained immunostimulatory cytokines IL-2 and IL-12, which have been shown to provide signals for dendritic cell and T- cell activation (Tawde et al. Vaccine. 30(38):5675-5681 (2012)). Finally, the vaccine polymer solution was spray-dryed through Buchi 191 Spray Dryer. The spray drying procedure aerosolizes the vaccine antigen-polymer matrix, where optimum water removal from the droplet results in nanoparticles containing the vaccine antigen in a polymer matrix (schematically represented in Fig. 1)).

Vaccine particles described herein were formulated using the ID8 cell lysate or SP17 protein (5%, w/w), β -cyclodextrin, ethyl cellulose, human or mouse plasma, CpG, HPMCAS, and targeting agent AAL dissolved in deionized water. Briefly, HPMCAS were dissolved in an alkaline solution. Human or mouse plasma was added to the polymeric solution at pH 7.0, which contains albumin (although albumin was not part of the basic particle system). AAL was added to the solution, followed by the addition of ID8 lysate or SP17 protein (5%, w/w). The CpG sequence #1826 was also included in this mixture. The weight ratio of CpG: nanoparticle polymer was 1 :500. This corresponds to a dose of 10 µg CpG per dose of nanoparticle vaccine (5 mg/mouse), which has been previously shown to provide an efficient adjuvant effect in oral vaccines formulations (McCluskie et al. Vaccine A9(4-5):413-422 (2000)). Finally, this aqueous solution was spray dried by one-step process using the Buchi B-191 mini spray dryer (Buchi Corporation, New Castle, Delaware) at inlet temperature 125° C, outlet temperature 80° C, 500 L/h, and 2% feed rate (20 mL/h) of peristaltic pump, and nozzle diameter of 0.7 mm (Akande at al. J Microencapsul .27(4):325-336 (2010)). The resulting particles can be stored at -20°C in a desiccant chamber till further use.

### Example 2

### Testing of Various Adjuvants as Potentiators of SP17 Nanoparticle Vaccine

In place or in addition to CpG, several different adjuvants can be used in the same particulate formulation. Inclusion of additional adjuvants can further stimulate the initial innate immunity response and thus ameliorate the vaccine efficacy. The activation of the immune response can be maximized by designing the molecular topology of the microparticle according to pathogen-associated molecular patterns (PAMPs). These patterns represent small molecular sequences, which are consistently found on pathogens (Mogensen TH, Clin Microbiol Rev. 22(2): 240-273 (2009)). Adjuvants mimic these PAMPs, thus improving the response to nanoparticle vaccines both in vivo and in vitro.

The following adjuvants have been screened as potentiators of nanoparticle oral vaccine formulation described in the present invention:
- AddaVax™(MF59) a squalene-based oil-in- water nano-emulsion
- Alhydrogel 2% an aluminium hydroxide adjuvant (referred herein to as alum) available as wet gel suspension.,
- Flagellin (FliC) a recombinant flagellin protein encoded by the fliC gene from Salmonella typhimurium.
- R848 an imidazoquinoline compound and agonist for TLR7 and TLR8
- MPLA (monophosphoryl lipid A), a TLR4 agonist that is a derivative of lipid A from Salmonella minnesota R595 lipopolysaccharide (LPS or endotoxin),
- ODN 1826 a group of synthetic oligodeoxynucleotides containing unmethylated CpG motifs (CpG ODNs).
- P4 a 28-30 kDa lipoprotein present in all typeable and nontypeable strains of H. influenzae.

For the screening of various adjuvants (described above 1-7, Table 1), nanoparticles were prepared as described above (Example 1). Cancer/testis antigen (CTA) SP17 was used as a target antigen. Briefly, 400,000 human monocyte-derived dendritic cells (DC) were incubated with 0.2 mg of vaccine nanoparticles (5 µg total protein) and 0.2 mg adjuvant nanoparticles (2 µg adjuvant). Dendritic cells were incubated with the nanoparticles for 24 hours. Following the incubation period, cells were washed with PBS and incubated with specific markers for flow-cytometry analysis. All adjuvant treated groups included 7 g of SP17 in macroparticles (MP) and 2 g of antigen in MP.

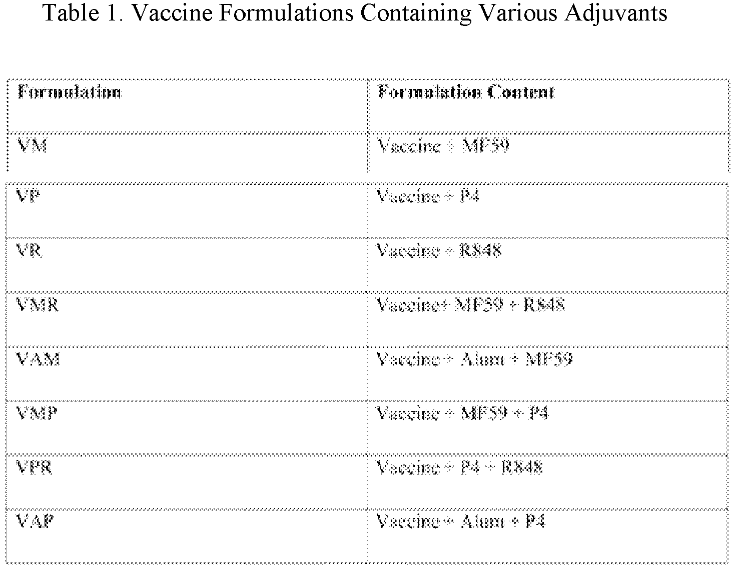

Nitric oxide is a non-specific marker of immune system activation. Concentrations of nitrite (N02) levels reflecting nitric oxide production in DCs can be measured with the Griess reagent system (Promega). As shown in Figure 2, most of the adjuvant treated groups showed a statistically significant (p< 0.05) increase in nitric oxide levels compared to the SP17 (SP) microparticles (MP) alone. Flagellm and MPLA were the only adjuvants that did not cause an increase in nitric oxide levels compared to SP17 MP alone. However, while Flagellin and MPLA inclusion did not cause an increase in nitric oxide levels in this specific example, it cannot be said that these adjuvants would not lead to immune system activation if tested using a different system (host,. Thus, the inventors anticipate that t inclusion of Flagellin and/or MPLA into the vaccine formulations of the present disclosure even in the absence of other adjuvants can lead to enhanced activation of the immune system. Furthermore, Flagellin and MPLA may contribute to the specific immune response such as upregulation of DC markers (see Example 4).

### Example 3

### Addition of Adjuvants to SP17 Nanoparticle Vaccine Enhances the Activation of Dendritic Cells

Signaling via CD80 and CD86 regulates dendritic cell activation and maturation. Given the results described in Example 2 and the observation that most of the formulations containing adjuvants led to nitric oxide release in DCs, the inventors sought to further evaluate the immune response following the incubation of DCs with microparticle formulations described in Table 1. Briefly, DCs were treated with microparticle formulations for 16 hours, where each group treated with adjuvants received 4 g of antigen and 1 g of the corresponding adjuvant(s) in microparticles. Following the 16-hour incubation period, cells were washed with PBS and evaluated for CD80 and CD86 marker expression using flow-cytometry analysis. As shown in Figures 3 and 4, both CD80 (Figure 3) and CD86 (Figure 4) expression was increased following the incubation of DCs with vaccine microparticles containing an adjuvant, compared to the vaccine alone. Furthermore, DCs exposed to formulations containing more than one adjuvant (VPR, VPM, and VMR) exhibited even higher expression of CD80 and CD86 compared to DCs treated with vaccine microparticles with a single adjuvant.

These experiments show that: (1) treatment of cells with vaccine microparticles along with an adjuvant results in the increased activation of DCs; and (2) inclusion of additional adjuvants (more than one) into the formulation further intensifies DC activation.

### Example 4

### Inclusion of an Adjuvant into Microparticle Formulations Leads to the Induction of ICAM-1 expression in DCs

Adhesion receptors such as Intracellular Adhesion Molecule (ICAM-1) (also known as CD54) are expressed on mature dendritic cells and function to promote adhesion to T cells. Next, the inventors tested the ability of DCs treated with SP17 microparticle formulations containing different adjuvants to upregulate ICAM-1 expression. Briefly, DC 2.4 cells were incubated with and without an adjuvant. All DCs treated with an adjuvant received 7 g SP17 antigen and 2 g of adjuvant in 1 mg of nanoparticle. As demonstrated in Figure 5, ICAM-1 expression was elevated in MPLA and P4 treated groups (p<0.001) compared to SP17 MP. [0084] Thus, in addition to the increase in CD80 and CD86 expression, inclusion of adjuvants in the microparticle formulations also increases ICAM-1 expression, which reflects conditions of DC maturation (the expression of ICAM-1 is low in immature DCs and increases upon maturation).

### Example 5

### Addition of Adjuvants to SPI 7 Nanoparticle Vaccine Leads to Increased CD40 and MHC II Expression in DCs

Next, expression of CD40 and MHC II were analyzed as stimulation markers of DCs. DCs were incubated with various microparticle formulations (Table 1) for 16 hours. All adjuvant-treated groups received 4 µg of antigen and 1 µg of adjuvant in 1 mg of nanoparticle. As shown in Figure 6, the pattern of CD40 and MHC II expression is similar to that observed for CD80 and CD86 (Example 3), where it was shown that CD80 and CD86 expression on DCs increases with the inclusion of additional adjuvants as compared to vaccine mp with a single adjuvant. Figure 6 demonstrates that CD40 and MHC II expression was elevated in cells receiving vaccine microparticle along with combination of adjuvants (p<0.05) compared to vaccine MP with a single adjuvant.

### Example 6

### Characterization of Physical and Biological Properties of Nanoparticles

The inventors analyzed the physical properties of the nanoparticles used in the foregoing experiments. The particle size, and zeta potential was determined using a Malvern Zeta Sizer. Initial studies focused on SP-17 loaded nanoparticles. Prototype studies produced batches with consistent size ranges of around 3.59 microns, which was in the desired particle size range of 1-5 microns (Fig. 7) and the zeta potential was around + 9.36 mV. Furthermore, scanning electron microscopy revealed that the particles are almost spherical but they have a rough surface.

Furthermore, the stability of the vaccine antigen upon encapsulation (following spray drying process) was evaluated in order to determine whether spray drying leads to degradation and/or instability of the vaccine antigen. Sodium dodecyl sulfate polyacrylamide gel electrophoresis (SDS-PAGE) analysis showed that the vaccine antigen remains stable on spray drying (Figure 8).

Most successful vaccines are generated by activation of dendritic cells via multiple Toll-like receptors (TLRs) to stimulate pro -inflammatory cytokines. The triggering of combinations of TLRs can induce synergistic production of cytokines leading to the enhanced T- cell response. The inventors next evaluated the immune response in terms of the release of nonspecific markers like nitric oxide (Figure 9). Dendritic cells were prepared from human peripheral blood monocytes as previously described (Chiriva-Internati et al. PLoS One. 5(5):el0471 (2010); Stagg et al. Methods Mo I Med. 64:9-22 (2001)). DCs were plated in a 96- well plate and after 2 hours SP17 antigen loaded nanoparticles were added to the cells. Blank nanoparticles and antigen in solution were used as controls. Nanoparticles or solution were allowed to be in contact with the dendritic cells for a period of 48 hours. After the 48-hour incubation period, the cell supernatant was removed and analyzed for nitrite (readout for nitric oxide) release (Fig. 9). As shown in Figure 9, incubation of human monocytes with SP17 microparticles led to a significant release of nitric oxide compared to the SP17 in solution. These findings indicate the activation of the innate immune response via treatment with SP17 antigen loaded particles in human dendritic cells.

### Example 7

### In Vivo Oral Vaccine Studies using Breast Cancer Animal Model

Recent studies using an oral vaccine against melanoma (D'Souza et al. J Drug Target. 20(2): 166-173 (2012)) and prostate cancer (Akalkotkar et al. J Drug Target. 20(4):338-346 (2012)) have demonstrated excellent tumor protection in mice receiving the formulation containing the M-cell targeting agent, Aleuria aurantia lectin (AAL) (Tawde et al. Vaccine. 30(38):5675-5681 (2012)).

In this Example, the inventors conducted oral nanoparticle vaccine efficacy and potency determination studies using the 67NR breast cancer model. Animals were primed with 5 mg of either blank or vaccine microparticle (equivalent to 250 µg of 67NR antigen) suspension in 200 of citrate buffer (10 mM, pH 4.0) using oral feeding needle. One week after the initial dose, two booster doses of same strength were given in alternate weeks.

After the final booster animals were challenged with IX 106 live murine 67NR tumor cells intra-peritoneally to determine the efficacy of 67NR microparticulate vaccine. Briefly, 67NR tumor cells were suspended in 100 µ 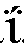 , of serum-free DMEM medium and injected subcutaneously into animals. Following 2 weeks (post-injection of tumor cells), vaccination resulted in significantly lower tumor volumes compared to the control (Fig 10).

In order to gain insight into the uptake of vaccine particles into Peyer's patches of animals following oral administration, the inventors conducted vaccine uptake studies using labeled biopolymer nanoparticles. Use of nanoparticles labeled with fluorescent dyes crystal violet (Figure 11A, left) and IR dye CW-800 (Figure 11B, right)) indicated excellent uptake of particles into the Peyer's patches. Furthermore, Figure 12 shows that oral nanoparticle vaccine described herein localized to the Peyer's patches.

Regarding the stability of the antigen-nanoparticle complexes, it is anticipated that 67NR tumor antigen-nanoparticle complexes exhibit stability qualitatively similar to that observed for another breast cancer antigen, 4T07 (Figure 13 taken from Chablani et al. J Pharm Sci. 101(10): 3661-71. (2012)). There, in the case of 4T07 antigen, dissolution studies demonstrated sustained release of antigen from the microparticles.

### Example 8

### Preparation of bio-degradable vaccine nanoparticles containing ovarian cancer cells lysate

The inventors prepared a bio-degradable vaccine nanoparticles containing ID8 ovarian cancer cell lysate (specifically lysate proteins or antigens) in addition to SP17 antigen.

### Preparation of tumor lysate and SP17 mouse protein:

The ID 8 ovarian cancer cells were cultured for 3 days in 75 cm² tissue culture flasks in a 5% C02, 37°C incubator until sub-confluent. The cells were washed with Phosphate Buffered Saline (PBS) pH 7.4 and scraped in the presence of hypotonic buffer. Subsequently, the cells were subjected to five freeze-thaw cycles, which includes freezing the cell suspension at - 80 °C for 10 minutes followed by thawing at 37 °C degrees for 10 minutes. The lysate was stored at -80 °C and used to prepare the vaccine. This is a standard protocol for the preparation of cell lysate and can be used to prepare cell lysates of various different cell types.

His-tagged recombinant SP17 mouse protein was expressed in E. coli and purified in the laboratory following standard protocols.

### Preparation of the vaccine nanoparticles:

The matrix system used for the microparticles of this oral vaccine composition contained β-cyclodextrin (60%), hydroxy propyl methyl cellulose acetate succinate (HPMCAS, 20%)) and ethyl cellulose (EC, 15%). Briefly, the biopolymers were dissolved in water together with the SP17 vaccine antigen (5% w/w). The vaccine formulation also contained antigens derived from the ID8 ovarian cancer cell lysate (10%), as well as M-cell targeting ligand Aleuria Aurantia lectin (AAL), that has been shown to improve the targeting of the particles to the Peyer's patches. In addition, immunostimulatory cytokines (IL-2 and IL-12), and CpG oligodeoxynucleotide were also included in the vaccine formulation. The vaccine formulation also contained mouse plasma. The vaccine polymer solution was spray dried through a Buchi B191 Spray Dryer (Buchi Corporation) and optimum water removal from the droplets resulted in nanoparticles containing the vaccine antigen and other components in a polymer matrix.

### Example 9

### Characterization of the ID8 and SP 17 Antigen Containing Vaccine Nanoparticles

The particle size and zeta potential were determined using a Zetatrac Ultra zeta potential and particle size analyzer (Figure 14). Average particle size of polymer microparticles was found to be 1.1 microns. The zeta potential of the microparticles was determined to be -31.8 mV. The antigen content after spray drying was assessed by SDS-PAGE analysis, which confirmed a stable SP17 content following spray drying. The particle vaccine formulation was highly stable with a maximum 60% release (data not shown). Furthermore, the morphology of the spray dried particles was determined by scanning electron microscopy (Figure 15).

The vaccine preparation was next evaluated to determine the release kinetics of the encapsulated material. The particulate vaccine formulations were suspended in simulated gastric fluid for 2 hours followed by intestinal fluid with the goal to mimic the conditions of the stomach and intestine. The vaccine antigen content wasdetermined by a micro BCA protein assay method (commercially available, Thermo Fisher Scientific Inc, Waltham, MA USA 02451). The nanoparticle vaccine formulation was highly stable, with no more than 55% of antigen released after 5 hours.

### Example 10

### In Vitro Biological Evaluation of the ID8 and SPI 7 Antigen Containing Vaccine Nanoparticles

Next, the best vaccine formulation was selected by taking the advantage of evaluating the innate immune response in vitro in dendritic cells. Thus, the immune response was measured in terms of the release of the non-specific marker nitric oxide by DCs. As a negative control, the inventors used either blank nanoparticles or antigen in solution. The nanoparticles or antigen solution were incubated with DCs for a period of 48 hours. After the incubation period, the cell supernatant was removed and analyzed for nitric oxide release. Incubation of DCs with SP17 microparticles showed a significant increase in nitric oxide release, indicating the activation of the innate immune system.

### Example 11

### In Vivo Biological Evaluation of the ID8 and SPI 7 Antigen Containing Vaccine Nanoparticles

C57/B16 mice (N=16) were injected with 2 x 10<6>ID8 cells intra-peritoneally. Control mice (N=4) were injected with PBS. Animal weight and signs of ascites were recorded every other day. After 37 days, mice with comparable tumors were divided into 4 groups (N=4/group) and the oral vaccination schedule was initiated (Table 2). Animals were boosted with the vaccine3 times, where each treatment followed the prior one by one week.

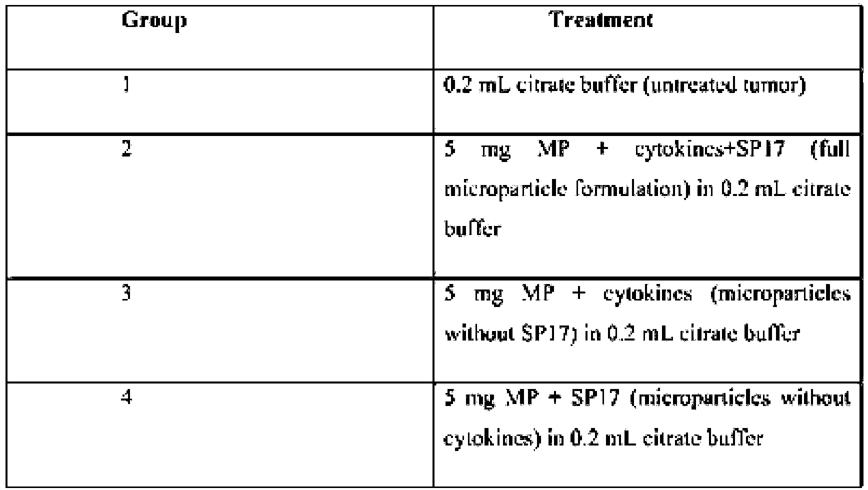

While these studies are ongoing, the data obtained up to this date demonstrated that ID8-injected mice had advanced stage tumors about 1 month after the injection (Figure 16 A), and that the vaccine, even without cytokine, conferred protection in terms of survival advantage (Figure 16 B). These results show a survival advantage conferred by an oral CTA-containing vaccine composition administered therapeutically even after a short time. It is anticipated that as the experiment continues and the data mature inclusion of cytokines will show a survival benefit or a reduction in tumor burden or both. (In humans, prolongation of survival can be assessed by reference to mean survival time from diagnosis).

### References cited

1. Chiriva-Internati M. Sperm protein 17: clinical relevance of a cancer/testis antigen, from contraception to cancer immunotherapy, and beyond. Inrernational reviews of immunology. Apr-Jun 2011 :30(2-3):138-149.
2. Chiriva-Internati M. Crizzi F. Wachtel MS, et al. Biological treatment for liver tumor and new potential biomarkers. Digestive diseases and sciences. Mar 2008;53(3):836-843.
3. Chiriva-Internati M, Grizzi F, Weidanz JA, et al. A NOD/SCID tumor model for human ovarian cancer that allows tracking of tumor progression through the biomarker Sp17. J Immunol Methods. Apr 10 2007;321(1-2):86-93.
4. Chiriva-Internati M, Mirandola L, Yu Y, et at. Cancer testis antigen, ropporin is a potential target for multiple myeloma immunotherapy. Journal of immunotherapy (Hagerstown, Md. : 1997). Jul-Aug 2011;34(6):490-499.
5. Chiriva-Internati M, Pandey A, Saba R, et al. Cancer testis antigens: a novel target in lung cancer. International reviews of immunology. Oct 2012;31 (5):321-343.
6. Chiriva-Intenati M, Wang Z, Pochopien S, Salati E, Lim SH. Identification of a sperm protein 17 CTL epitope restricted by HLA-A1. Int J Cancer. Dec 10 2003;107(5).863-865.
7. Chiriva-Internati M. Wang Z, Salati E, Bumm K, Barlogie B, Lim SH. Sperm protein 17 (Spl7) is a suitable target for immunotherapy of multiple mycloma. Blood. Aug 1 2002; 100(3),961-965.
8. Chiriva-Internati M, Wang Z, Salati E, Wroblewski D. Lim SH. Successful generation of sperm protein 17 (Spl7)-specifie cytotoxie T lymphocytes from normal donors: implication for tumour-specific adoptive immunotherapy following allogeneic stem cell transplantation for Sp17-positive multiple mycloma, Scandinavian Journal of immunology. Oct 2002;56(4):429-433.
9. Chiriva-Internati M, Weidanz JA, Yu Y, et al. Sperm protein 17 is a suitable target for adoptive T-cell-based immunotherapy in human ovarian cancer. Journal of immunotherapy (Hager, Md. : 1997). Oct 2008:31(8):693-703.
10. Chiriva-Internati M, Yu Y, Mirandola L, et al. Identification of AKAP-4 as a new cancer/testis antigen for detection and immunotherapy of prostate cancer. The Prostate. Jan 2012;72(1):12-23.
11. Chiriva-Internati M, Yu Y, Mirandola L, et al. Cancer testis antigen vaccination affords long-term protection in a murine model of ovarian cancer. PLoS One. 2010;5(5):c10471.
12. Grizzi F, Franceschini B, Hamrick C, Frezza EE, Cobos E, Chiriva-Intermati M. Usefulness or cancer-testis antigens as biomarkers for the diagnosis and treatment of hepatocellular carcinoma. Journal of translational medicine, 2007;5:3.
13. Grizzi F, Gactani P, Franceschini B. et al. Sperm protein 17 is expressed in human nervous system tumours. BMC Cancer, 2006;6:23.
14. Lim SH, Wang Z, Chiriva-Internati M, Xue Y. Sperm protein 17 is a novel cancer-testis antigen in multiple mycloma Blood. Mar 1 2001;97(5):1508-1510
15. Mirandola L, M JC, Cobos E, ct al. Cancer testis antigens: novel biomarkers and targetable proteins for ovarian cancer. International reviews of immunology Apr-Jun 2011 ;30(2-3); 127-137.
16. Pandey A, Kurup A, Shrivastava A. et al. Cancer testes antigens in breast cancer: biological rote, regulation, and therapeutic applicability. International reviews of immunology. Oct 2012;31(5):302-320.
17. Straugh JM, Jr., Shaw DR, Guerrero A, et al. Expression of sperm protein 17 (Sp17) in ovarian cancer. Int J Cancer. Mar I 2004; 108(6):805-811.
18. Wang Z, Zhang Y. Liu H, Salati E. Chiriva-Internati M. Lim SH. Gene expression and immunologic consequence of SPAN-Xb in myeloma and other hematologic malignancies. Blood. Feb I 2003;101(3):955-960.
19. Chiriva-Intemati M, Cobos E, Cannon MJ. Prospects and challenges for immunotherapy of ovarian cancer-what can we learn from the tumor microenvironment? Int Rev Immunol. Apr-Jun 2011;30(2-3):67-70.
20. Chirive-Internati M. Cobos E, Da Silva DM. Kast WM. Sperm fibrous sheath proteins: a potential new class of target antigens for use in human therapeutic cancer vaccines Cancer Immun. 2008;8:8.
21. Chiriva-Internati M. Cobos E. Kast WM. Advances in immunotherapy of multiple mycloma: from the discovery of tumor-associated antigens to clinical trials. Int Rev Immunol May-Aug 2007;26(3-4): 197-222.
22. ChirivaInternati M. Cagliano N, Donetti E, ct al. Sperm protein 17 is expressed in the sperm fibrous sheath. J Transl Med. 2009;7:61.
23. Chiriva-Internati M, Grizzi F, Franceschini B, et al. Is sperm protein 17 a useful target for tumor immunotherapy? Blood. Sep 15 2003:102(6):2308-2309.
24. Chiriva-Internati M. Wang. Z., Xue Y, Bumm K, Hahn AB. Lim SH, Sperm protein 17 (Spl7) in multiple myeloma: opportunity for mycloma-specific donor T cell infusion to enhance grafl-versus-myeloma effect without inercasing graft-versus-host discase risk. Eur J Immunol. Aug 2001-31(8):2277-2283.
25. Bot A, Chiriva-Internati M. Topics in this issue: cancer testes antigens, immune checkpoint, inflammation associated with ischemia-reperfusion and integrin targeting. Int Rev Immunol. Oct 2012;31(5):299-30L
26. Grizzi F. Chirive-Internati M. Translating sperm protein 17 as a target for immunotherapy from the bench to the bedside in the light of cancer complexity. Tissue Antigens. Feb 2013;81(2):116-118
27. Chiriva-Internati M, Ferrari R, Yu Y. et al. AKAP-4; a novel cancer testis antigen for multiple mycloma. Br J Haematol. Feb 2008.140(4):465-468.
28. Mirandola L, Yu Y, Jenkins MR. ct al. Tracking human multiple mycloma xenografts in NOD-Rag-I/IL-2 receptor gamma chain-null mice with the novel biomarker AKAP-4. BMC Cancer, 2011; 11:394.
29. Chiriva-Internati M. Ferrari R, Prabhakar M. ct al. The pituitary tumor transforming gene 1 (PTTG-1): an immunological target for multiple mycloma. J Transl Med. 2008;6: 15.
30. Grizzi F. Franceschini B. Musardo S. Frezza EE, Cobos E, Chiriva-Internati M. Pituitary tumor transforming gene I in the Liver. Hepatology. Dec 2006;44(6):1701-1702.
31. Chiriva-Internati M. Mirandola L, Kast WM, Jenkins MR, Cobos E, Cannon MJ. Understanding the cross-talk between ovarian tumors and immune cells: mechanisms for effective immunotherapies. Int Rev Immunol. Apr-Jun 2011;30(2-3):71-86.
32. Chiriva-Internati M, Wang Z, Salati E, Timmins P, Lim SH. Tumor vaccine for ovarian carcinoma targeting sperm protein 17. Cancer. 2002;94(9):2447-2453.
33. Alalawi R, Kim M, Mirandola L, ct al. New Antigens In Non-Small Cell Lung Cancer Detected Both In Serology And Tissue. Chest. 2011; doi:10.1378/chest.1118730.
34. Malik B, Goyal AK, Mangal S, Zakir F, Vyas SP. Implication of gut immunology in the design of oral vaccines Curr Mol Med. Feb 2010;10(1):47-70.
35. Shen H. Ackerman AL, Cody V, et al. Enhanced and prolonged cross-presentation following endosomal escape of exogenous antigens encapsulated in biodegradable nanoparticles. Immunology. Jan 2006; 117(1):78-88.
36. Akande J. Yeboah KG, Addo RT, Siddig A. Oettinger CW, D'Souza MJ. Targeted delivery of antigens to the gut-associated lymphoid tissues: 2. Ex vivo evaluation of lectin-labelled albumin microspheres for targeted delivery of antigens to the M-cells of the Peyer's patches. J Microencapsul. 2010;27(4):325-336.
37. Pasetti MF, Simon JK, Sztein MB, Levine MM. Immunology of gut mucosal vaccines. Immunol Rev. 2011;239(1): 125-148.
38. Devriendt B, De Geest BG, Cox E. Designing oral vaccines targeting intestinal dendritic cells. Expert Opin Drug Deliv. 2011;8(4):467-483.
39. Azizi A, Kumar A, Diaz-Mitoma F, Mestecky J. Enhancing oral vaccine potency by targeting intestinal M cells. PLoS Pathog. 2010:6(11):1001147.
40. Kim SH. Seo KW, Kim J, Lee KY, Jang YS. The M cell-targeting ligand promotes antigen delivery and induces antigen-specific immune responses in mucosal vaccination. J Immunol. 2010:185(10);5797-5795.
41. Emtage PC, Wan Y, Muller W, Graham FL, Gauldie J. Enhanced interleukin-2 gene transfer immunotherapy of breast cancer by coexpression of B7-1 and B7-2. J Interferon Cytokine Res. Nov 1998; 18(11):927-937.
42. Zhang XQ, Dahle CE, Baman NK, Rich N, Weiner GJ, Salem AK. Potent antigen- specific immune responses stimulated by codelivery of CpG ODN and antigens in degradable microparticles. J Immunother. Jul-Aug 2007;30(5):469-478.
43. Chiriva-Internati M. Yu Y. Mirandola L. et al.. Cancer testis antigen vaccination affords long-term protection in a murine model of ovarian canccr. PLoS One. 2030;5(5).
44. Tawde SA, Chablani L, Akalkolkar A, et al. Formulation and evaluation of oral microparticulate ovarian cancer vaccines Vaccine. 2012;30(33):5675-5681,
45. McCluskie MJ, Davis HL. Oral. intrarectal and intranasal immunizations using CpO and non-CpG oligodeoxynucleotides as adjuvants. Vaccine. 2000:19(4-5):413-422.
46. Uddin AN, Bejugam NK, Gayakwad SG, Akther P, D'Souza MJ. Oral delivery of gastro- resistant microencapsulated typhoid vaccine. J Drug Target. Aug 2009; 17(7):553-560.
47. Yaboah KG, D'Souza M J. Evaluation of albumin microspheres as oral delivery system for Mycobacterium tuberculosis vaccincs. J Microencapsul. Mar 2009;26(2):166-179.
48. Akalkotkar A, Tawde SA, Chablani L, D'Souza MJ. Oral delivery of particulate prostate cancer vaccine: in vitro and is vivo evaluation, J Drug Target. May 2012;20(4):338-346.
49. Bernadetta. DS. Microparticulate delivery system for protein and vaccine therapy, Ph.D Thesis, Mercer Unviersity 2011.
50. Stagg AJ, Burke F, Hill S, Knight SC. Isolation of mouse spleen dendritic cells. Methods Mol Med. 2001;64:9-22.
51. D'Souza B, Bhowmik T, Shashidharamurthy R, Oettinger C, Selvaraj P, D'Souza M. Oral microparticulate vaccine for melanoma using M-cell targeting, J Drug Target, Feb 2012;20(2):166-173.

## Claims

1. An oral anticancer vaccine composition comprising spray-dried microparticles comprising β-cyclodextrin, hydroxypropylmethyl cellulose acetate succinate (HPMCAS), ethyl cellulose, recombinant SP17 protein, *aleuria aurantia* lectin (AAL) in a microfold cell (M-cell) targeting effective amount and CpG in an innate immune response stimulatory amount,
the β-cyclodextrin, hydroxypropylmethyl cellulose acetate succinate (HPMCAS) and ethyl cellulose comprised in a gastric acid resistant, enteric erodable matrix, and
the recombinant SP17 protein being encapsulated in the particles and provided in an amount within the range from 1 to 10% w/w based on the weight of the particles.

2. The composition of claim 1, wherein the microparticles have an average diameter based on particle volume within the range from 1 to 5 µm.

3. The composition of claim 1 or 2, wherein the matrix comprises 60% beta cyclodextrin, 20% HPMCAS and 15% ethyl cellulose.

4. The composition of any preceding claim, wherein the recombinant SP17 protein and the AAL are embedded in the matrix.

5. The composition of any preceding claim, wherein the recombinant SP17 protein is a full-length recombinant SP17 protein.

6. The composition of any preceding claim, wherein the microparticles comprise tumor lysate providing a lysate protein content (as measured by BCA assay) to the particles within the range from 1 to 10 % (w/w) based on the weight of the particles.

7. The composition of any preceding claim, wherein the AAL is provided in an amount within the range from 0.1 to 0.5% (w/w) based on the weight of the particles.

8. The composition of any preceding claim, wherein the CpG is provided in an amount within the range from 0.1 to 0.5% (w/w) based on the weight of the particles.

9. The composition of any preceding claim, further comprising at least one immune response stimulating cytokine that has the property of stimulating at least one of a Thl immune response and a Th2 immune response.

10. The composition of claim 9, wherein the at least one cytokine is selected from the group consisting of interleukin 2 (IL-2), interleukin 12 (IL-12) or both.

11. The composition of any preceding claim, for use in the vaccination of a subject affected with any of:
(a) a malignant tumor selected from the group of solid tumors and hematologic malignancies;
(b) a solid tumor selected from the group consisting of ovarian cancer, hepatocellular carcinoma, breast cancer, and prostate cancer;
(c) a hematologic malignancy selected from the group consisting of multiple myeloma, acute myelogenous leukemia, acute lymphoblastic leukemia, chronic lymphocytic leukemia, chronic myelogenous leukemia, hairy cell leukemia;
(d) ovarian cancer.

12. The composition for use of claim 11, for:
(a) repeated administration at least two and up to 10 times at an interval of 1 to 3 days after the preceding administration; and
(b) administration to a subject in an amount within the range between 5mg and 320 mg said composition containing from 0.1 to 0.5 % (w/w) of AAL based on the weight of the particles.

## Patentansprüche

1. Orale Antikrebs-Impfstoffzusammensetzung, umfassend sprühgetrocknete Mikropartikel, die β-Cyclodextrin, Hydroxypropylmethylcelluloseacetatsuccinat (HPMCAS), Ethylcellulose, rekombinantes SP17-Protein, Aleuria-Aurantia-Lectin (AAL) in einer für Mikrozellen (M-Zellen) wirksamen Menge und CpG in einer die angeborene Immunantwort stimulierenden Menge umfassen,
wobei das β-Cyclodextrin, das Hydroxypropylmethylcelluloseacetatsuccinat (HPMCAS) und die Ethylcellulose in einer magensäureresistenten, im Darm auflösbaren Matrix beinhaltet sind, und
das rekombinante SP17-Protein in den Partikeln eingekapselt ist und in einer Menge im Bereich von 1 bis 10 Gew.-% (w/w), auf Grundlage des Gewichts der Partikel, vorgesehen ist.

2. Zusammensetzung nach Anspruch 1, wobei die Mikropartikel einen durchschnittlichen Durchmesser auf Grundlage des Partikelvolumens innerhalb des Bereichs von 1 bis 5 µm aufweisen.

3. Zusammensetzung nach Anspruch 1 oder 2, wobei die Matrix 60 % Beta Cyclodextrin, 20 % HPMCAS und 15 % Ethylcellulose umfasst.

4. Zusammensetzung nach einem der vorangegangenen Ansprüche, wobei das rekombinante SP17-Protein und das AAL in die Matrix eingebettet sind.

5. Zusammensetzung nach einem der vorangegangenen Ansprüche, wobei das rekombinante SP17-Protein ein in voller Länge rekombinantes SP17-Protein ist.

6. Zusammensetzung nach einem der vorangegangenen Ansprüche, wobei die Mikropartikel Tumorlysat umfassen, das den Partikeln einen Lysatproteingehalt (gemessen mittels BCA-Assay) im Bereich von 1 bis 10 Gew.-% (w/w), auf Grundlage des Gewichts der Partikel, bereitstellt.

7. Zusammensetzung nach einem der vorangegangenen Ansprüche, wobei das AAL in einer Menge im Bereich von 0,1 bis 0,5 Gew.-% (w/w), auf Grundlage des Gewichts der Partikel, bereitgestellt wird.

8. Zusammensetzung nach einem der vorangegangenen Ansprüche, wobei das CpG in einer Menge im Bereich von 0,1 bis 0,5 Gew.-% (w/w), auf Grundlage des Gewichts der Partikel, bereitgestellt wird.

9. Zusammensetzung nach einem der vorangegangenen Ansprüche, die zumindest ein eine Immunantwort stimulierendes Zytokin umfasst, das die Eigenschaft hat, zumindest eine von einer Th1-Immunantwort und einer Th2-Immunantwort zu stimulieren.

10. Zusammensetzung nach Anspruch 9, wobei das mindestens eine Zytokin ausgewählt ist aus der Gruppe bestehend aus Interleukin 2 (IL-2), Interleukin 12 (IL-12) oder beiden.

11. Zusammensetzung nach einem der vorangegangenen Ansprüche, zur Verwendung bei der Impfung eines Individuums, das von einem der Folgenden betroffen ist:
(a) einem bösartigen Tumor, ausgewählt aus der Gruppe der soliden Tumore und hämatologischen Malignitäten;
(b) einem festen Tumor, ausgewählt aus der Gruppe bestehend aus Eierstockkrebs, hepatozellulärem Karzinom, Brustkrebs und Prostatakrebs;
c) ein hämatologisches Malignom, ausgewählt aus der Gruppe bestehend aus multiplem Myelom, akuter myelogener Leukämie, akuter lymphatischer Leukämie, chronischer lymphatischer Leukämie, chronischer myelogener Leukämie, Haarzellenleukämie,
(d) Eierstockkrebs.

12. Zusammensetzung zur Verwendung nach Anspruch 11, für:
(a) wiederholte Verabreichung zumindest zweimal und bis zu zehnmal in einem Abstand von 1 bis 3 Tagen nach der vorangegangenen Verabreichung; und
(b) Verabreichung an ein Individuum in einer Menge im Bereich zwischen 5 mg und 320 mg, wobei die Zusammensetzung 0,1 bis 0,5 Gew.-% (w/w) AAL, auf Grundlage des Gewichts der Partikel, enthält.

## Revendications

1. Composition de vaccin anticancéreux orale comprenant des microparticules séchées par pulvérisation comprenant de la β-cyclodextrine, de l'acétosuccinate d'hydroxypropylméthyl cellulose (HPMCAS), de l'éthyl cellulose, une protéine SP17 recombinante, de la lectine *aleuria aurantia* (AAL) dans une quantité efficace pour cibler les cellules à plis microscopiques (cellules M) et du CpG dans une quantité stimulante de réponse immunitaire innée,
la β-cyclodextrine, l'acétosuccinate d'hydroxypropylméthyl cellulose (HPMCAS), et l'éthyl cellulose étant compris dans une matrice érodable entérique résistante à l'acide gastrique, et
la protéine SP17 recombinante étant encapsulée dans les particules et fournie dans une quantité dans la plage de 1 à 10 % p/p sur la base du poids des particules.

2. Composition selon la revendication 1, dans laquelle les microparticules ont un diamètre moyen sur la base d'un volume de particules dans la plage de 1 à 5 µm.

3. Composition selon la revendication 1 ou 2, dans laquelle la matrice comprend 60 % de bêta-cyclodextrine, 20 % de HPMCAS et 15 % d'éthyl cellulose.

4. Composition selon l'une quelconque des revendications précédentes, dans laquelle la protéine SP17 recombinante et l'AAL sont incorporées dans la matrice.

5. Composition selon l'une quelconque des revendications précédentes, dans laquelle la protéine SP17 recombinante est une protéine SP17 recombinante de pleine longueur.

6. Composition selon l'une quelconque des revendications précédentes, dans laquelle les microparticules comprennent un lysat tumoral fournissant une teneur en protéines de lysat (mesurée par dosage BCA) aux particules dans la plage de 1 à 10 % (p/p) sur la base du poids des particules.

7. Composition selon l'une quelconque des revendications précédentes, dans laquelle l'AAL est fournie dans une quantité dans la plage de 0,1 à 0,5 % (p/p) sur la base du poids des particules.

8. Composition selon l'une quelconque des revendications précédentes, dans laquelle le CpG est fourni dans une quantité dans la plage de 0,1 à 0,5 % (p/p) sur la base du poids des particules.

9. Composition selon l'une quelconque des revendications précédentes, comprenant en outre au moins une cytokine de stimulation de réponse immunitaire qui a la propriété de stimuler au moins l'une d'une réponse immunitaire Th1 et d'une réponse immunitaire Th2.

10. Composition selon la revendication 9, dans laquelle l'au moins une cytokine est choisie dans le groupe consistant en l'interleukine 2 (IL-2), l'interleukine 12 (IL-12), ou les deux.

11. Composition selon l'une quelconque des revendications précédentes, pour une utilisation dans la vaccination d'un sujet atteint de l'une quelconque parmi :
(a) une tumeur maligne choisie dans le groupe des tumeurs solides et des malignités hématologiques ;
(b) une tumeur solide choisie dans le groupe consistant en le cancer de l'ovaire, le carcinome hépatocellulaire, le cancer du sein, et le cancer de la prostate ;
(c) une malignité hématologique choisie dans le groupe consistant en le myélome multiple, la leucémie myélogène aigue, la leucémie lymphoblastique aigue, la leucémie lymphoïde chronique, la leucémie myélogène chronique, la leucémie à tricholeucocytes ;
(d) le cancer de l'ovaire.

12. Composition pour une utilisation selon la revendication 11, destinée à :
(a) une administration répétée au moins deux et jusqu'à 10 fois à un intervalle de 1 à 3 jours après l'administration précédente ; et
(b) une administration à un sujet dans une quantité dans la plage entre 5 mg et 320 mg de ladite composition contenant 0,1 à 0,5 % (p/p) d'AAL sur la base du poids des particules.
